(19) Europäisches Patentamt
European Patent Office
Office européen des brevets

(11) Publication number: **0 242 134 A2**

(12) **EUROPEAN PATENT APPLICATION**

(21) Application number: **87303132.2**

(22) Date of filing: **10.04.87**

(51) Int. Cl.[4]: **C 07 D 487/04**, A 61 K 31/40 // (C07D487/04, 209:00, 205:00)

(30) Priority: **15.04.86 US 852069**

(43) Date of publication of application: **21.10.87 Bulletin 87/43**

(84) Designated Contracting States: **AT BE CH DE ES FR GB GR IT LI LU NL SE**

(71) Applicant: **MERCK & CO. INC., 126, East Lincoln Avenue P.O. Box 2000, Rahway New Jersey 07065-0900 (US)**

(72) Inventor: **Cama, Lovji D., 80 Morningside Avenue, Creskill New Jersey 07626 (US)**
Inventor: **Heck, James V., 961 Nepawin Lane, Scotch Plains New Jersey 07076 (US)**

(74) Representative: **Hesketh, Alan, Dr. et al, European Patent Department Merck & Co., Inc. Terlings Park Eastwick Road, Harlow Essex, CM20 2QR (GB)**

(54) **N-amino quaternized heteroarylium carbapenem antibacterial agents.**

(57) Carbapenems having the

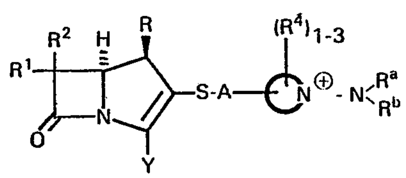

are useful antibacterial agents.

EP 0 242 134 A2

3417P/1166A
3438P/1166A



TITLE OF THE INVENTION

N-AMINO QUATERNIZED HETEROARYLIUM CARBAPENEM ANTIBACTERIAL AGENTS

BACKGROUND OF THE INVENTION

The present invention relates to antibacterial agents of the carbapenem class, in which the terminus of the "SR" sidechain at the 2-position is characterized by an N-amino quaternized heteroarylium, as described in more detail further below.

Thienamycin was an early carbapenem antibacterial agent having a broad spectrum; it has the following formula:

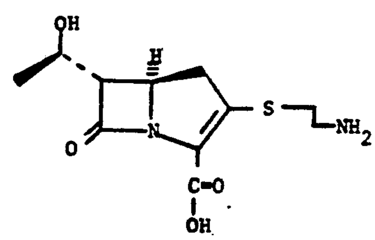

Later, N-formimidoyl thienamycin was discovered; it has the formula:

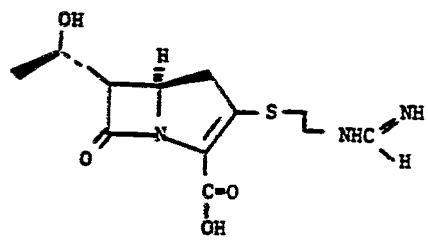

The N-amino quaternized heterarylium carbapenems of the present invention have an antibacterial spectrum and potency equal to or greater than, in most cases, that of either thienamycin or N-formimidoyl thienamycin. The N-amino quaternized heteroarylium carbapenems of the present invention are also more resistant than thienamycin or N-formimidoyl thienamycin to degradation by the dehydropeptidase enzyme DHP-I, thus permitting greater therapeutic application of the compounds.

More recently, carbapenem antibacterial agents have been described which have a 2-substituent which is a quaternized nitrogen-containing aromatic heterocycle; they have the formula:

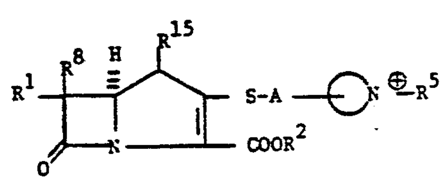

and are more fully described in GB 2 128 187A. However, the quaternizing substituent $R^5$ is not amino or substituted amino, as is the case with the carbapenem compounds of the present invention.

SUMMARY OF THE INVENTION

The present invention provides novel carbapenem compounds of the formula:

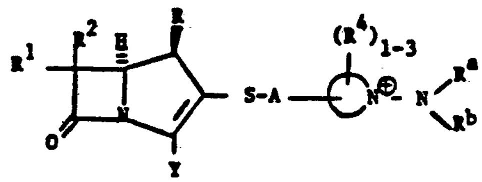

(I.)

wherein:

R is H or $CH_3$;

$R^a$ and $R^b$ are independently hydrogen, $C_1$-$C_4$ straight or branched alkyl, or $C_3$-$C_6$ cycloalkyl;

$R^1$ and $R^2$ are independently H, $CH_3$-, $CH_3CH_2$-, $(CH_3)_2CH$-, $HOCH_2$-, $CH_3CH(OH)$-, $(CH_3)_2C(OH)$_, $FCH_2$-, $F_2CH$-, $F_3C$-, $CH_3CH(F)$-, $CH_3CF_2$-, or $(CH_3)_2C(F)$-;

$R^4$ is independently selected from :

a) a trifluoromethyl group;

b) a halogen atom;

c) an unsubstituted or substituted $C_1$-$C_4$ alkoxy radical;

d) a hydroxy group;

e) an unsubstituted or substituted ($C_1$-$C_6$ alkyl) carbonyloxy radical;

f) a carbamoyloxy radical which is unsubstituted or substituted on nitrogen with one or two $C_1$-$C_4$ alkyl groups;

g) a $C_1$-$C_6$ alkylthio radical, $C_1$-$C_6$ alkylsulfinyl radical or $C_1$-$C_6$ alkylsulfonyl radical, each of which is unsubstituted or substituted on the alkyl group;

h) a sulfamoyl group which is unsubstituted or substituted on nitrogen by one or two $C_1$-$C_4$ alkyl groups;

i) an amino group;

j) a mono ($C_1$-$C_4$ alkyl) amino or di($C_1$-$C_4$ alkyl)amino group, each of which is unsubstituted or substituted on the alkyl group;

k) a formylamino group;

l) an unsubstituted or substituted ($C_1$-$C_6$ alkyl)carbonylamino radical;

m) a ($C_1$-$C_4$ alkoxy) carbonylamino radical;

n) a ureido group in which the terminal nitrogen is unsubstituted or substituted with one or two $C_1$-$C_4$ alkyl groups;

o) a ($C_1$-$C_6$ alkyl)sulfonamido group;

p) a cyano group;

q) a formyl or acetalized formyl radical;

r) an unsubstituted or substituted ($C_1$-$C_6$ alkyl)carbonyl radical wherein the carbonyl is free or acetalized;

s) an unsubstituted or substituted phenylcarbonyl or heteroarlycarbonyl radical;

t) a hydroximinomethyl radical in which the oxygen or carbon atom is optionally substituted by a $C_1$-$C_4$ alkyl group;

u) a ($C_1$-$C_6$ alkoxy)carbonyl radical;

v) a carbamoyl radical which is unsubstituted or substituted on nitrogen by one or two $C_1$-$C_4$ alkyl groups;

w) an N-hydroxycarbamoyl or N($C_1$-$C_4$ alkoxy)carbamoyl radical in which the nitrogen atom may be additionally substituted by a $C_1$-$C_4$ alkyl group;

x) a thiocarbamoyl group;

y) an amidino group 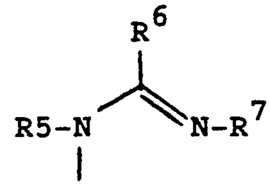 or

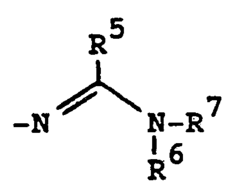

where $R^5$, $R^6$ and $R^7$ are independently hydrogen, $C_1$-$C_4$alkyl or wherein two of the alkyl groups together form a $C_2$-$C_6$alkylidene

radical optionally interrupted by a heteroatom and joined together to form a ring;

z) a carboxamidino group 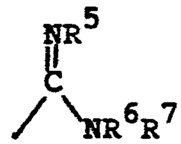 , where $R^5$, $R^6$ and $R^7$ are as defined above;

aa) a quanidinyl group where $R^6$ in ab) above is $NR^8R^9$ and $R^8$ and $R^9$ are as defined for $R^5$ through $R^7$ above;

ab) hydrogen;

ac) an unsubstituted or substituted $C_1$-$C_6$ alkyl radical;

ad) an unsubstituted or substituted $C_1$-$C_6$ alkenyl radical;

ae) an unsubstituted or substituted $C_1$-$C_6$ alkynyl radical;

af) a $C_3$-$C_7$ cycloalkyl radical in which the ring is substituted or unsubstituted and one or more atoms may be replaced by a heteroatom;

ag) a $C_3$-$C_7$ cycloalkyl methyl radical in which the ring may be substituted and one or more atoms may be replaced by a heteroatom;

ah) an unsubstituted or substituted $C_5$-$C_7$ cycloalkenyl radical;

ai) an unsubstituted or substituted phenyl or heteroaryl radical;

aj) an unsubstituted or substituted phenyl ($C_1$-$C_4$ alkyl) or heteroaryl ($C_1$-$C_4$ alkyl) radical; and

ak) an acidic side-chain of the structure $-B$ or $-(CH_2)_n-X-(CH_2)_m-W-B$ where:

n is 0-4;

m is 0-4;

X is $CHR^s$, CH=CH, phenylene $(-C_6H_4-)$, NH, $N(C_1-C_4$ alkyl), O, S, S=O, C=O, $SO_2$, $SO_2NH$, $CO_2$, CONH, $OCO_2$, OC=O, NHC=O; $R^s$=H, $O(C_1-C_4$ alkyl), $NH_2$, $NH(C_1-C_4$ alkyl), $N(C_1-C_4$ alkyl$)_2$, CN, $CONH_2$, $CON(C_1-C_4$ alkyl$)_2$, $CO_2H$, $SO_2NH_2$, or $SO_2NH(C_1-C_4$ alkyl);

W is a single bond, NH, $N(C_1-C_4$ alkyl), O, or S;

B is an acidic function selected from carboxy $(CO_2H)$, phosphono $[P=O(OH)_2]$, alkylphosphono $P=O(OH)-[C(C_1-C_4$ alkyl)] , alkylphosphinyl $[P=O(OH)-(C_1-C_4$ alkyl)], substituted phosphoramido $[P=O(OH)NH(C_1-C_4$ alkyl) and $P=O(CH)NHP^x]$, sulfino $(SO_2H)$, sulfo $(SO_3H)$, 5-tetrazolyl $(CN_4H)$, arylsulfonamido $(SO_2NHR^x)$ and acylsulfonamides selected from the structures $CONHSO_2(C_1-C_4$ alkyl), $CONHSO_2N(C_1-C_4alkyl)_2$, $SO_2NHCO(C_1-C_4alkyl)$ and $SO_2NHCOR^x$, where

$R^x$ is aryl or heteroaryl;

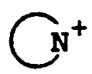 is a mono-or bicyclic quaternary heteroaryl group having 5-11 ring atoms of which, in addition to the quaternary nitrogen, up to four may be heteroatoms independently selected from N, S, or O;

A is a covalent bond or a bridging group selected from $-(CH_2)_{1-4}S-$; $-(CH_2)_{1-4}-O-$; $-(CH_2)_{1-4}-X-(CH_2)_{1-4}$ where X=O, S, NH, or $N(C_1-C_6)$alkyl; substituted or unsubstituted $C_1-C_4$ straight, $C_1-C_6$ branched or $C_3-C_7$ cycloalkyl groups wherein the substituents are selected from $C_1-C_6$ alkyl, $O-C_1-C_6$ alkyl, $S-C_1-C_6$ alkyl, halo, OH, $CF_3$, CN, $NH_2$, $NHC_1-C_6$ alkyl, $N(C_1-C_6$ alkyl$)_2$, $CO_2H$, $CONH_2$, CONH $(C_1-C_6$ alkyl), and $CON(C_1-C_6$ alkyl$)_2$; and

Y is selected from:
i) COOH or a pharmaceutically acceptable ester or salt thereof,
ii) $COOR^3$ wherein $R^3$ is a readily removable carboxyl protecting group,
iii) COOM wherein M is an alkali metal, or
iv) $COO^-$; provided that when Y is other than iv) a counterion $Z^-$ is provided.

With reference to the above definitions, "aryl" means phenyl or naphthyl; and "heteroaryl" means a mono- or bicyclic hydrocarbon in which up to four of the carbon atoms have been replaced by a heteroatom independently selected from N, S, and O.

It is preferred that when one of $R^1$ or $R^2$ is H, the other is $CH_3CH(OH)$-or $CH_3CH(F)$-, and $CH_3CH(OH)$- is most preferred.

Representative A groups are $-CH_2-$, $-CH(CH_3)-$, $-CH(C_2H_5)-$, $-(CH_2)_{2-4}$, $-CH(CH_3)-CH_2-$, $CH_2-CH(OCH_3)-$, $-CH(CH_3)-(CH_2)_2-$, $-CH(CH_2OH)-CH_2-$, $-CH(CF_3)-CH_2-$, $-CH_2-CH_2-S$, $-CH_2-CH_2-O$, $-(CH_2)_2-S-CH_2-$, $-(CH_2)_2-O-CH_2$, a single covalent bond, and the like.

A preferred A group is a substituted or unsubstituted $C_1$-$C_6$ linear or branched chain alkyl. A more preferred A group is $-CH_2-$, $-CH(CH_3)-$ or $(CH_2)_2-$.

Representative $R^4$ groups are $-CH_3$, $CH_2SO_3H$, $-CH_2CH_3$, $-(CH_2)_3CH_3$, $-OCH_3$, 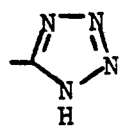, $-COOH$, $-OH$, $-CH_2OH$, $-CH_2COOH$, $-CH_2CH_2COOH$, $-CH_2CONH_2$, $-CH_2CH_2S^{\oplus}(CH_3)_2$, $-CH_2CH_2SO_3H$, 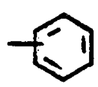, $-CONH_2$, $-SO_2NH_2$, $-SO_3H$, $-N(CH_3)_2$ $-CON(CH_3)_2$, $-CH_2N(CH_3)_2$, $-CH_2CN$, $-CH_2SCH_3$, $-CH_2SOCH_3$, $-CH_2SO_2CH_3$, $-SO_2CH_3$, $-SOCH_3$, $-CH_2OCH_3$, 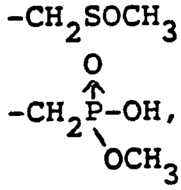, $-CF_3$, $-CH_2O\overset{O}{\overset{\|}{C}}NH_2$, $-CH_2SO_2NH_2$, Br, Cl, and F.

$R^a$ and $R^b$ are preferably selected from H and $CH_3$.

Useful examples of the 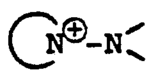 moiety are the following:

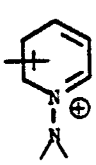 , 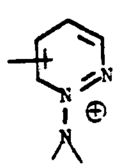 , 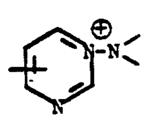 , 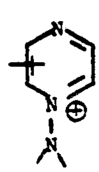 , 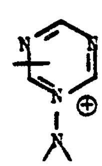 ,

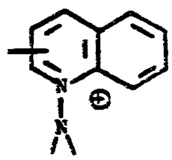 , 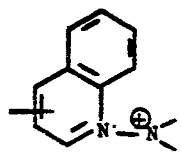 , 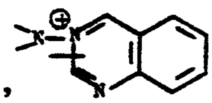 ,

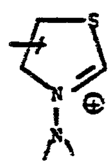 , 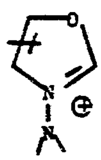 , 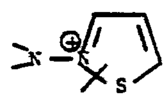 , 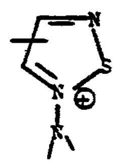 ,

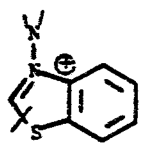 , 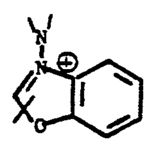 ,

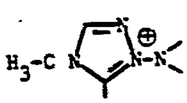 , 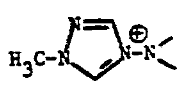 , 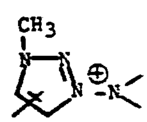 , 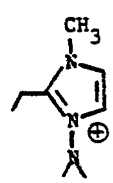 ,

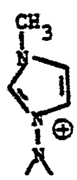 , 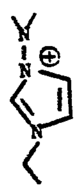 , 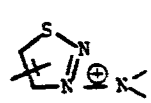 , 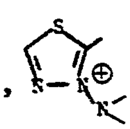 ,

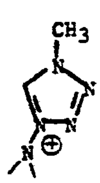 , 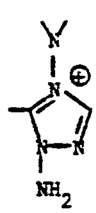 , 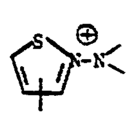

With regard to the entire compound of the present invention, and especially 2-position substituent:

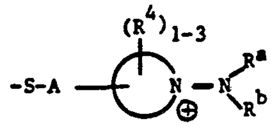

, the following are preferred:

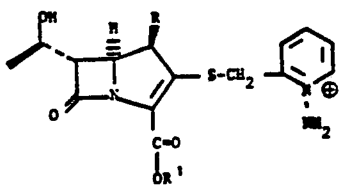

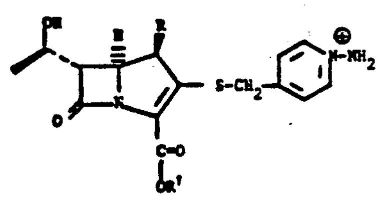

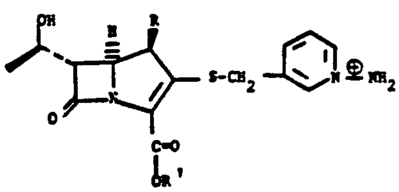

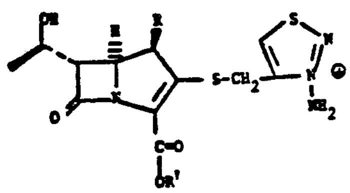

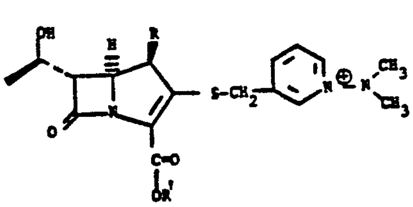

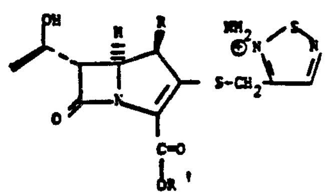

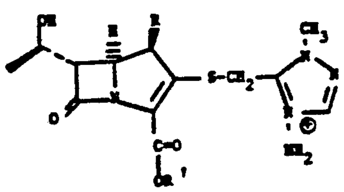

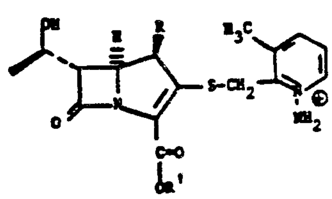

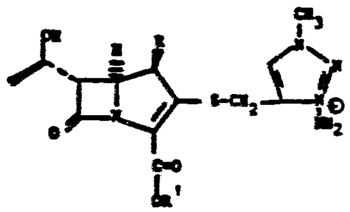

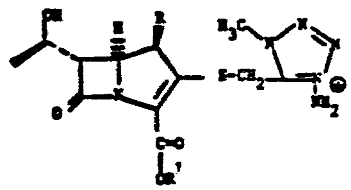

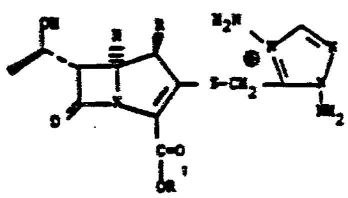

where R = H or $CH_3$;
X = Br, Cl, I or alkyl or arylsulfonate; and
R' = H, or a readily removable carboxyl protecting group.

The carbapenem compounds of the present invention are useful per se and in their pharmaceutically acceptable salt and ester forms in the treatment of bacterial infections in animal and human subjects. Conveniently, pharmaceutical compositions may be prepared from the active ingredients in combination with pharmaceutically acceptable carriers. Thus, the present invention is also concerned with pharmaceutical compositions and methods of treating bacterial infections utilizing as an active ingredient the novel carbapenem compounds of the present invention.

The pharmaceutically acceptable salts referred to above include non-toxic acid addition salts. The Formula I compounds can be used in the

form of salts derived from inorganic or organic acids. Included among such salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Also, the basic nitrogen-containing groups can be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others. Water or oil-soluble or dispersible products are thereby obtained.

The pharmaceutically acceptable esters of the novel carbapenem compounds of the present invention are such as would be readily apparent to a medicinal chemist, and include, for example, those described in detail in the U.S. Pat. No. 4,309,438, Column 9, line 61 to Column 12, line 51, which is incorporated herein by reference.

The novel carbapenem compounds of the present invention may also take the form where Y is $COOR^3$, where $R^3$ is a readily removable carboxyl

protecting group. Such conventional blocking groups consist of known ester groups which are used to protectively block the carboxyl group during the synthesis procedures described further below. These conventional blocking groups are readily removable, i.e., they can be removed, if desired, by procedures which will not cause cleavage or other disruption of the remaining portions of the molecule. Such procedures include chemical and enzymatic hydrolysis, treatment with chemical reducing agents under mild conditions, and catalytic hydrogenation. Examples of such ester protecting groups include benzhydryl, P-nitrobenzyl, 2-naphthylmethyl, allyl, benzyl, trichloroethyl, silyl such as trimethyl silyl, phenacyl, p-methoxybenzyl, acetonyl, o-nitrobenzyl, 4-pyridylmethyl, and $C_1$-$C_6$ alkyl such as methyl, ethyl or t-butyl. Included within such protecting groups are those which are hydrolyzed under physiological conditions, such as pivaloyloxymethyl, acetoxymethyl, phthalidyl, indanyl and methoxymethyl.

The compounds of the present invention are valuable antibacterial agents active against various Gram-positive and Gram-negative bacteria and accordingly find utility in human and veterinary medicine. Representative pathogens which are sensitive to antibiotics I include: *Staphylococcus aureus*, *Escherichia coli*, *Klebsiella Pneumoniae*, *Bacillus subtilis*, *Salmonella typhosa*, *Pseudomonas* and *Bacterium proteus*. The antibacterials of the invention are not limited to utility as medicaments; they may be used in all manner of industry, for example: additives to animal feed, preservation of food, disinfectants, and in other industrial systems

where control of bacterial growth is desired. For example, they may be employed in aqueous compositions in concentrations ranging from 0.1 to 100 parts of antibiotic per million parts of solution in order to destroy or inhibit the growth of harmful bacteria on medical and dental equipment and as bactericides in industrial applications, for example in waterbased paints and in the white water of paper mills to inhibit the growth of harmful bacteria.

The compounds of this invention may be used in any of a variety of pharmaceutical preparations. They may be employed in capsule, powder form, in liquid solution, or in suspension. They may be administered by a variety of means; those of principal interest include: topically or parenterally by injection (intravenously or intramuscularly).

Compositions for injection, a preferred route of delivery, may be prepared in unit dosage form in ampules, or in multidose containers. The compositions may take such forms as suspensions, solutions, or emulsions in oily or aqueous vehicles, and may contain formulatory agents. Alternatively, the active ingredient may be in powder form for reconstitution, at the time of delivery, with a suitable vehicle, such as sterile water. Topical applications may be formulated in hydrophobic or hydrophilic bases as ointments, creams, lotions, paints, or powders.

The dosage to be administered depends to a large extent upon the condition and size of the subject being treated as well as the route and frequency of administration -- the parenteral route by injection being preferred for generalized

infections. Such matters, however, are left to the routine discretion of the therapist according to principles of treatment well known in the antibiotic art. In general, a daily dosage consists of from about 5 to about 600 mg of active ingredient per kg of body weight of the subject in one or more treatments per day. A preferred daily dosage for adult humans lies in the range of from about 10 to 240 mg of active ingredient per kg of body weight. Another factor influencing the precise dosage regimen, apart from the nature of the infection and peculiar identity of the individual being treated, is the molecular weight of the chosen species of this invention.

The compositions for human delivery per unit dosage, whether liquid or solid, may contain from 0.1% to 99% of active material, the preferred range being from about 10-60%. The composition will generally contain from about 15 mg to about 1500 mg of the active ingredient; however, in general, it is preferable to employ a dosage amount in the range of from about 250 mg to 1000 mg. In parenteral administration, the unit dosage is usually the pure compound I in sterile water solution or in the form of a soluble powder intended for solution.

The preferred method of administration of the Formula I antibacterial is parenteral by i.v. infusion, i.v. bolus, or i.m. injection.

For adults, 5-50 mg of Formula I antibacterial per kg of body weight given 2, 3, or 4 times per day is preferred. Preferred dosage is 250 mg to 1000 mg of the Formula I antibacterial given two (b.i.d.) three (t.i.d.) or four (q.i.d.) times

per day. More specifically, for mild infections, and particularly urinary tract infections, a dose of 250 mg t.i.d. or q.i.d. is recommended. For moderate infections against highly susceptible gram positive and gram negative organisms, a dose of 500 mg t.i.d. or q.i.d. is recommended. For severe, life-threatening infections against organisms at the upper limits of sensitivity to the antibiotic, a dose of 1000 t.i.d. or q.i.d. is recommended.

For children, a dose of 5-25 mg/kg of body weight given 2, 3, or 4 times per day is preferred; a dose of 10 mg/kg t.i.d. or q.i.d. is usually recommended.

Antibacterial compounds of Formula I are of the broad class known as carbapenems or 1-carbadethiapenems. Certain of these carbapenems are susceptible to attack by a renal enzyme known as dehydropeptidase (DHP). This attack or degradation may reduce the efficacy of the carbapenem antibacterial agent. Inhibitors of DHP and their use with carbapenem antibacterial agents are disclosed in the prior art [see published European Patent Applications No. 79102616.4 filed July 24, 1979 (Patent Number 10573); 79102615.6, filed July 24, 1979 (application no. 15573); and No. 82107174.3, filed August 9, 1980 (application no. 72014)].

The compounds of the present invention may, where DHP inhibition is desired or necessary, be combined or used with the appropriate DHP inhibitor as described in the aforesaid published applications. Thus, to the extent that the cited European patent applications 1.) define the procedure for determining DHP susceptibility of the present carbapenems and 2.)

disclose suitable inhibitors, combination compositions and methods of treatment, they are incorporated herein by reference. A preferred weight ratio of Formula I compound:DHP inhibitor in the combination compositions is about 1:1. A preferred DHP inhibitor is 7-(L-2-amino-2-carboxyethylthio)-2-(2,2-dimethyl-cyclopropanecarboxamide)-2-heptenoic acid or a useful salt thereof.

These combination compositions and their use are further embodiments of the present invention.

DETAILED DESCRIPTION OF THE INVENTION

The N-amino quaternized heterarylium carbapenem compounds of the present invention may be prepared in accordance with well known procedures in the art. Particularly useful are the following synthetic schemes in which the symbols R, $R^1$, $R^2$, A and 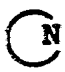 are as defined above:

A. Preparation of the 2-Position Side Chain

Scheme 1.

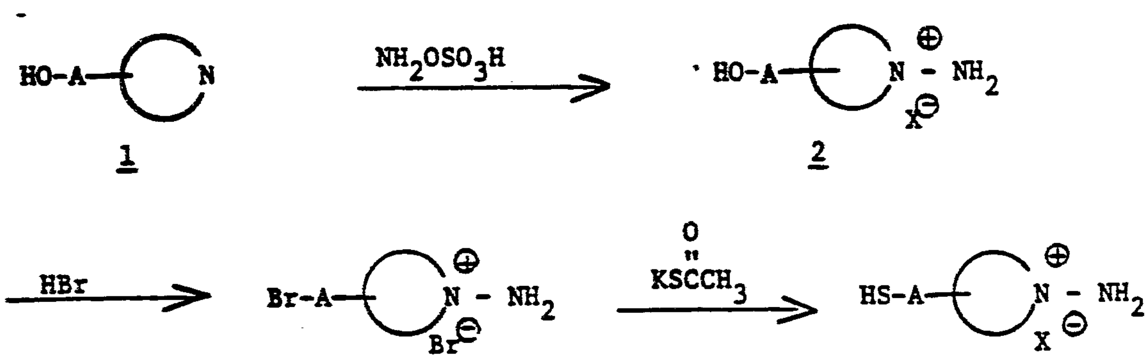


1 2

3 4

Scheme 2.

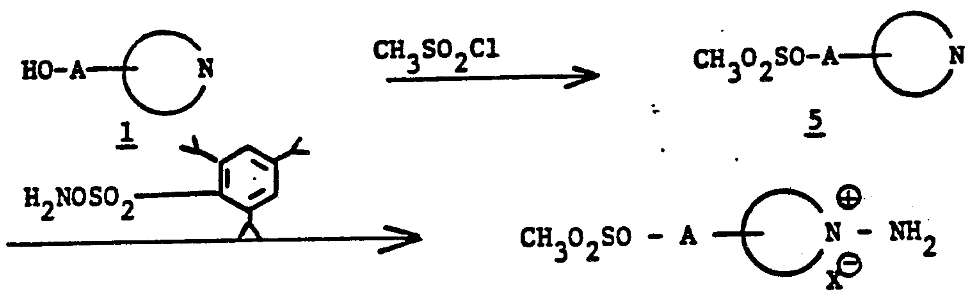


1 5 6

B. Substitution of the N-Amino Group

Scheme 1.

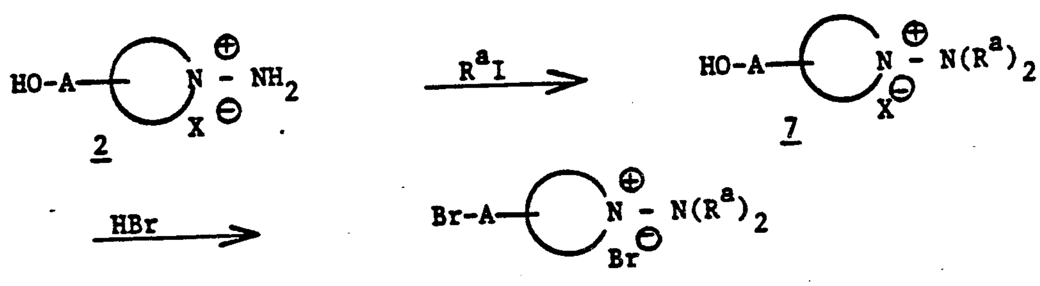


2 7 8

Scheme 2.

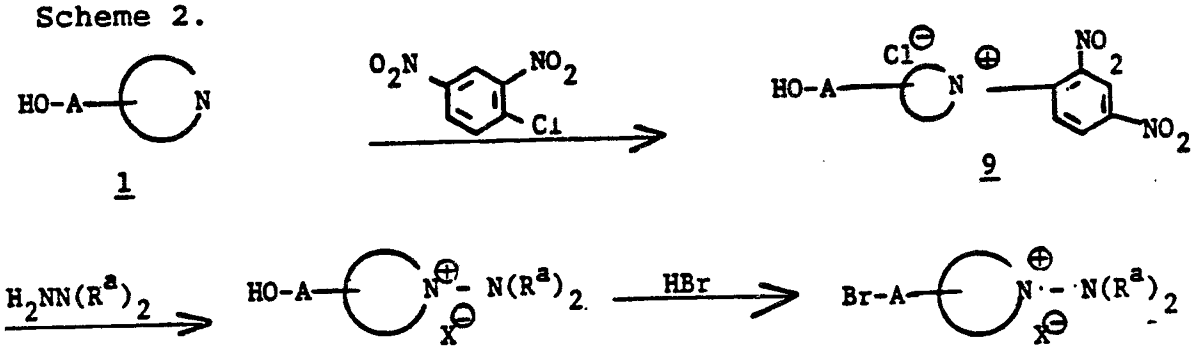


1 9 10 8

C. Preparation of Carbapenems

Scheme 1.

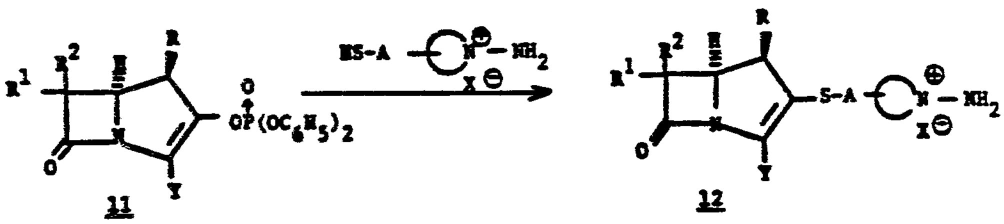


11 12

(Y=protected carboxyl, e.g. COOPNB)

Scheme 2.

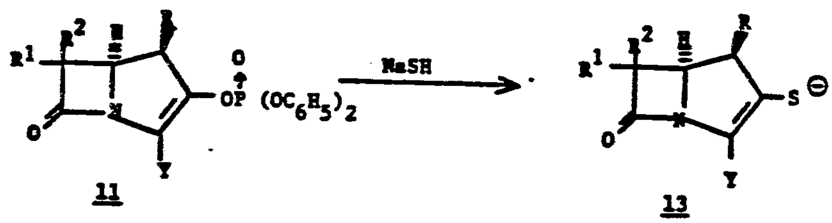


11 13

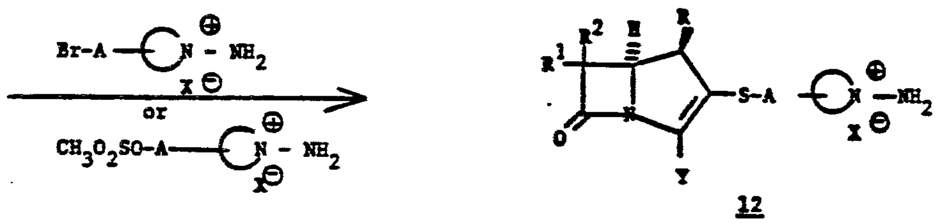


12

Scheme 3.

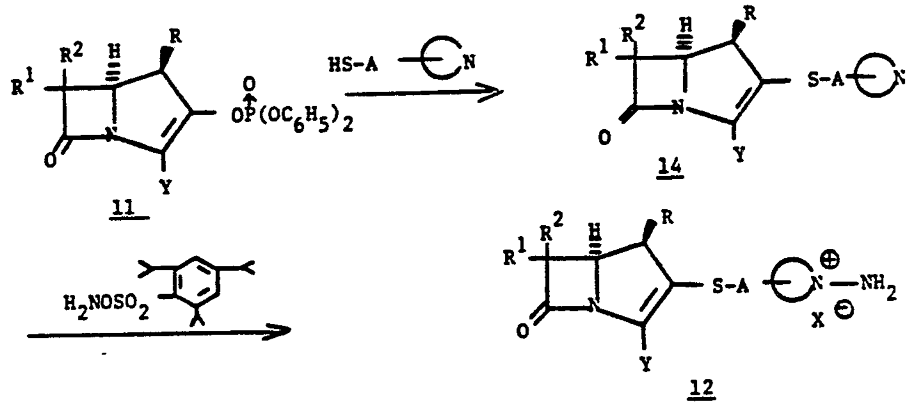

11 14 12

D. Deblocking

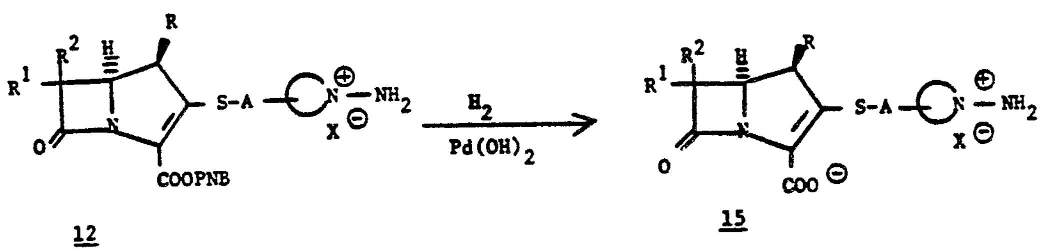


12 15

A. Preparation of the 2-Position Side Chain
Scheme 1.

The starting material, such as 2-pyridylcarbinol, is dissolved in water buffered to a pH of 7, and after heating to a temperature of 70°-90°C., preferably 80°C., hydroxylamine-O-sulfonic acid is added in the presence of potassium carbonate. The quaternizing N-amino substituent on the 2-position side chain is derived from the hydroxylamine-O-sulfonic acid. The carbinol portion of the starting material is then converted to a thiol group by first reacting it with hydrobromic acid, preferably 48%, under heating at 85°-120°C., preferably 90°C., for from 12-48 hrs., preferably 24 hrs., followed by treatment with a thioacetate, preferably potassium thioacetate, at -10°-10°C., preferably 0°C., then room temperature, for from 1-2 hours preferably 1.5 hours. Both steps are carried out under a nitrogen atmosphere. The thiol reactant thus formed is used to prepare the N-amino heteroarylium final product as shown under C., Scheme 1.

A more detailed description of these procedures may be found in Example 1, Steps A through C.

Scheme 2

An alternative to preparation of the 2-position side chain as described above may be found in treating the same carbinol starting material with methanesulfonyl chloride in the presence of triethylamine. The reaction is carried out in a solvent such as methylene chloride at a temperature of -10°-10°C., preferably 0°C., which rises to room

temperature while the reaction mixture is stirred for 0.5-2 hours, preferably 1 hour. The procedure just described converts the carbinol portion of the starting material to a methanesulfonyl (mesylate) group, which is analagous to the conversion of the carbinol portion to a bromo group as detailed in Scheme 1 above. The mesylate reactant thus formed is used to prepare the N-amino heteroarylium final product as shown under C., Scheme 2. The quaternizing N-amino substituent is added by treating a solution of e.g., 4-pyridylcarbinol mesylate, in a suitable solvent such as dichloromethane at a temperature of -10°-10°C., preferably 0°C., with 0-(2,4,6-triisopropylbenzenesulfonyl)hydroxylamine. The reaction mixture is allowed to stand at room temperature for from 2-4, preferably 3 hours.

A more detailed description of these procedures may be found in Example 7, Steps A and B.

B. Substitution of the N-Amino Group Scheme 1.

The procedures described immediately above are concerned with addition of the quaternizing N-amino substituent. This amino group may, in turn, be substituted by $C_1$-$C_4$ straight or branched alkyl or $C_3$-$C_6$ cycloalkyl, in accordance with the following procedure. The N-amino quaternized heteroarylium, e.g., N-aminopyridiniumcarbinol bisulphate, dissolved in a suitable solvent such as ethanol, is treated with the desired alkyliodide, e.g., methyl iodide, in the presence of potassium carbonate or sodium carbonate and the reaction mixture is heated at 55°-75°C., preferably 65°C.,

for 10-20, preferably 15 hours. The treatment is then repeated until the alkylation is complete.

The substituted amino reactant in the carbinol form prepared as described immediately above is next converted to the bromo form by dissolving it in 48% hydrobromic acid and heating at 70°-90°C., preferably 80°C., preferably 15 hours. The bromo reactant thus formed is used to prepare the N-substituted amino heteroarylium final product as shown under C., Scheme 2.

A more detailed description of these procedures may be found in Example 4, Steps A and B.

Scheme 2.

N-Aminoheteroaryliumalkyl bromides required for the enethiolate alkylation described in Section C, Scheme 2 may also be prepared as described in Section B, Scheme 2. The heteroaralkyl carbinol 1 is quaternized by exposure to 2,4-dinitrochlorobenzene in a solvent such as acetonitrile or acetone at a temperature of 0°-80°C. The resulting salt 9 is treated with hydrazine or a mono- or 1,1-disubstituted hydrazine in a solvent such as water, ethanol or dioxane at a temperature of 0°-100°C to afford aminoquaternary salt 10. The alcohol function is converted to the requisite bromide 8 most conveniently by treatment of 10 with hydrogen bromide in water or acetic acid at a temperature of 80°-120°C. See Chem. Pharm. Bull., 19, 130-138 (1971).

C. Preparation of Carbapenems

Scheme 1.

In this procedure for preparing the N-amino quaternized heteroarylium carbapenem final product, the carbapenem nucleus with a diphenylphosphoryloxy group at the 2-position is reacted with the N-amino heteroarylium side chain group in the form of a thiol. The carbapenem nucleus is dissolved in a suitable solvent such as tetrahydrofuran (THF), DMF, DMAC, dioxane etc. and cooled to -25°-5°C., preferably -15°C., under nitrogen. The thiol reactant, e.g., N-amino-2-pyridiniummethylthio chloride, is dissolved in a suitable solvent such as water, DMAC, DMF etc. and added to the reaction mixture, followed by dropwise addition of diisopropylethylamine.

The diphenylphosphoryloxy starting material may be prepared in accordance with the following known procedures: (1) where R=H: Salzmann et al., JACS, 102: 6161(1980); and (2) where R=$CH_3$: Shih et al., Heterocycles, 21: 29(1984).

A more detailed description of these procedures may be found in Example 1, Step D.

Scheme 2.

In this alternative procedure, the carbapenem nucleus as the diphenylphosphoryloxy compound is dissolved in a suitable solvent such as N,N-dimethylacetamide (DMAC) or DMF and cooled to -30°--10°C., preferably -17°C., under nitrogen, and sodium hydrogen sulfide, preferably dissolved in the same solvent, is then added dropwise, followed by diisopropylethylamine. The reaction mixture is

stirred for 0.5-1.0, preferably 0.75 hour at -25°-10°C., preferably -17°C., to give the sulfide form of the nucleus, which is in turn reacted with the 2-sidechain substituent in the form of a bromo compound, e.g., N-amino-3-bromomethylpyridinium chloride. The reaction mixture is stirred for 0.5-1.5, preferably 1.0 hour, at -25° - -10°C., preferably -17°C.

A more detailed description of these procedures may be found in Example 3, Step C.

Scheme 3.

In this alternative procedure the N-amino quaternizing substituent is added after the 2-position sidechain has been added to the carbapenem nucleus.

D. Deblocking

Deblocking of the carboxyl protecting group, e.g., 4-nitrobenzyl (PNB), is readily accomplished with the use of 20% palladium hydroxide/carbon, or 10% Pd/carbon with the reaction mixture being hydrogenated at 30-50, prepferably 40 p.s.i. for 1.0 - 1.5, preferably 1.25 hours.

The following examples illustrate the preparation of the compounds of Formula I in accordance with the synthesis schemes outlined above.

EXAMPLE 1

(5R,6S)-2-(N-amino-2-pyridiniummethylthio)-6-(1R-hydroxyethyl)-carbapen-2-em-3-carboxylate

Step A. N-amino-2-pyridiniumcarbinol chloride

2-Pyridylcarbinol (4.4 g, 0.04 mol) is dissolved in water (20 ml) and 1M pH7 phosphate buffer (20 ml). Potassium carbonate (5.5 g, 0.04 mol) is added and the mixture is heated to 80°. Hydroxylamine-O-sulfonic acid (4.52 g, 0.04 mol) is added in small batches over 15 minutes and the mixture is stirred for another 15 minutes. The reaction is followed by HPLC and further quantities of potassium carbonate and hydroxylamine-O-sulfonic acid are added until the reaction is complete. The reaction mixture is evaporated to a small volume and the precipitated inorganic salts are filtered off. The residual filtrate is placed on a column of Dowex 50 resin (acid cycle) and eluted with water until neutral and then with 4N HCl until the effluent shows no UV absorption at 240 nm. The HCl eluted fractions are collected and evaporated to near dryness, dissolved in ethanol, filtered and evaporated to give the desired product as a pale yellow solid (1.95 g). NMR(200 MHz, $D_2O$)d 5.04(s, $ArCH_2O$), 7.85(dd, 5-pyr H), 8.08(d, 3-pyr H), 8.36(dd, 4-pyr H), 8.71(d, 6-pyr H).

Step B. N-amino-2-bromomethylpyridinium chloride

N-amino-2-pyridiniumcarbinol chloride (0.5 g) is dissolved in 48% HBr (2 ml) and heated under nitrogen at 90° for 24 hours. The HBr is removed under reduced pressure and the residue is taken up in

hot acetonitrile and allowed to crystallize to give the desired product (0.440g).
NMR(200 MHz, $D_2O$)δ 5.02(s, $ArCH_2Br$), 8.0(dd, 5-pyr H), 8.2(d, 3-pyr H), 8.44(dd, 4-pyr H), 8.89(d, 6-pyr H).

Step C. N-amino-2-pyridiniummethylthiol chloride

N-amino-2-pyridiniummethylbromide chloride (0.41 g) is dissolved in methanol (4 ml) and cooled to 0° under nitrogen. Potassium thioacetate (0.230 g, 1.1 eq) is added and the mixture is stirred at room temperature for 90 minutes. The mixture is cooled to 0° and treated with acetyl chloride (0.14 ml) and then allowed to stir at room temperature overnight. The solvent is removed under reduced pressure to give a solid residue which is the desired product.
NMR(200 MHz, $D_2O$)δ 4.26(s, $ArCH_2S$), 7.92(dd, 5-pyr H), 8.12(d, 3-pyr H), 8.4(dd, 4-pyr H), 8.81(d, 6-pyr H).

Step D. (5R,6S)-2-(N-amino-2-pyridiniummethylthio)-6-(1R-hydroxyethyl)carbapen-2-em-3-carboxylate

4-Nitrobenzyl-(5R,6S)-2-diphenylphosphoryloxy-6(1R-hydroxyethyl)-carbapen-2-em-3-carboxylate (58 mg, 0.1 mmol) is dissolved in THF (2 ml) and cooled to -15° under nitrogen. The thiol from Step C (17 mg, 0.1 mmol) in water (0.5 ml) is added followed by the dropwise addition of diisopropylethylamine (18 µl, 0.1 mmol). The reaction mixture is followed by UV and further quantities of the thiol and diisopropylamine are added until the reaction has proceeded to at least 50%. The reaction mixture is

diluted with N-methylmorpholine pH7 buffer (1.25 ml, 0.5 M), water (5 ml), ethyl acetate (2.5 ml) and n-butanol (5 ml). 20% Palladium hydroxide/carbon (60 mg) is added and the mixture is hydrogenated at 40 pounds pressure for 1.25 hours. The catalyst is filtered off and washed with water, the filtrate and washings are extracted with ethyl acetate and the aqueous phase is evaporated to 5 ml and placed on a Dowex 50 resin column ($Na^+$ cycle) and eluted with water. The fractions containing the product are detected by UV, collected, and evaporated to a small volume and freeze dried to give the desired product (3.3 mg).
NMR(200 MHz, $D_2O$)δ 1.31(d, 6.5Hz, C-8 $CH_3$), 3.2(m, C-1 H), 3.46(dd, C-6 H), 4.24(m, C-5 H, C-8 H),7.96(dd, 5-pyr H), 8.3(d, 3-pyr H), 8.42(dd, 4-pyr H), 8.88(d, 6-pyr H). UV ($H_2O$, nm) λmax 270,290(sh). IR (nujol) $cm^{-1}$.1765

## EXAMPLE 2

(1R,5R,6S)-1-Methyl-2-(N-amino-2-pyridiniummethylthio)-6-(1R-hydroxyethyl)-carbapen-2-em-3-carboxylate

Starting with 4-nitrobenzyl-(1R,5R,6S)-1-methyl-2-diphenylphosphoryloxy-6-(1R-hydroxyethyl)-carbapen-2-em-3-carboxylate and following the procedure of Example 1, Step D, one obtains the title compound (20%). NMR(200 MHz, $D_2O$)δ 1.2(d, 7Hz, C-1 $CH_3$), 1.31(d, 6.5Hz, C-8 $CH_3$), 3.46(m, C-1 H), 3.52(dd, C-6 H), 4.24(dd, C-5 H), 7.98(m, 5 and 3-pyr H), 8.4(dd, 4-pyr H), 8.85(d, 6-pyr H). UV ($H_2O$,nm,) λmax 272,290(sh).

## EXAMPLE 3

(1R,5R,6S)-1-Methyl-2-(N-amino-3-pyridiniummethylthio)-6-(1R-hydroxyethyl)-carbapen-2-em-3-carboxylate

Step A. N-amino-3-pyridiniumcarbinol chloride

Starting with 3-pyridylcarbinol and reacting it with hydroxylamine-O-sulfonic acid as described in Example 1, Step A one obtains the title compound. NMR(200 MHz, $D_2O$)δ 4.91(s, $ArCH_2O$), 8.02(dd, 5-pyr H), 8.36(d, 4-pyr H), 8.7(d, 6-pyr H), 8.79(s, 2-pyr H). MS FAB m/e 125.

Step B. N-amino-3-bromomethylpyridinium chloride

The product of Step A is carried through the reaction conditions of Example 1, Step B to give the title compound. NMR(200 MHz, $D_2O$)δ 4.76(s, $ArCH_2Br$), 8.0(dd, 5-pyr H), 8.43(d, 4-pyr H), 8.7(d, 6-pyr H), 8.88(s, 2-pyr H). MS FAB, m/e 187, 189.

Step C. (1R,5R,6S)-1-methyl-2-(N-amino-3-pyridinium-methylthio)-6-(1R-hydroxyethyl)-carbapen-2-em-3-carboxylate

4-nitrobenzyl-(1R,5R,6S)-1-methyl-2-diphenyl-phosphoryloxy-6-(1R-hydroxyethyl)-carbapen-2-em-3-carboxylate (58 mg, 0.1 mmol) is dissolved in N,N-dimethylacetamide (DMAC) (0.5 ml) and cooled to -17° under nitrogen. NaSH (6 mg in 0.5 ml DMAC) is added dropwise followed by diisopropylethylamine (DIEA) (18 µl, 0.1 mmol). The reaction is allowed to stir 45 minutes at -17° and the bromo compound (22.3 mg, 0.1 mmol) in 0.5 ml DMAC is added followed by a further 18 µl of DIEA and the reaction mixture is stirred

at -17° for one hour. The reaction mixture is diluted with buffer, water, ethyl acetate and n-butanol and hydrogenated using palladium hydroxide catalyst and purified as described in Example 1, Step D, to give the desired product (14 mg). NMR(200 MHz, $D_2O$)δ 1.2(d, j=6.5 Hz, C-1 $CH_3$), 1.27(d, j=7Hz, C-8 $CH_3$), 3.32 (m, C-1 H), 3.48(dd, C-6 H), 4.2(m, C-5 H, C-8 H), 7.96(dd, 5-pyr H), 8.34(d, 4-pyr H), 8.65(d, 6-pyr H), 8.78(s, 2-pyr H). UV ($H_2O$, nm) λmax 250,298.

## EXAMPLE 4

(1R,5R,6S)-1-methyl-2-(N,N-dimethylamino-3-pyridinium-methylthio)-6-(1R-hydroxyethyl)-carbapen-2-em-3-carboxylate

Step A. 3-N-(N,N-dimethylamino)-pyridiniumcarbinol bisulfate

N-amino-pyridiniumcarbinol bisulphate (0.6 g) is dissolved in ethanol (5 ml) and treated with methyl iodide (5 ml) and potassium carbonate (1 g). The reaction is heated at 65° overnight. The solid is filtered off and washed with ethanol. The filtrate and washings are evaporated to dryness and taken up in ethanol (5 ml) and treated with methyliodide (4 ml) and sodium carbonate (1 g) and the reaction is again heated to 65° overnight. The reaction mixture is filtered and the residue is washed with ethanol, the filtrate and washings are evaporated under reduced pressure to give the crude desired product which is used for conversion to the bromo compound in the next step. NMR(200 MHz, $D_2O$)δ 3.21(s, N-Me) 5.0(s, $ArCH_2O$), 8.2(dd,

5-pyr H), 8.61(d, 4-pyr H), 9.12(d, 6-pyr H), 9.18(s, 2-pyr H).

Step B. <u>3-N-(N,N-dimethyl)amino-bromomethylpyridinium chloride</u>

The product from the previous step is dissolved in 48% HBr (2 ml) and heated at 80° overnight under nitrogen. The reaction mixture is evaporated to dryness and the residue is taken up in acetonitrile (10 ml) and stirred with saturated NaCl solution containing sodium thiosulfate (100 mg). The aqueous phase is separated and the organic phase is evaporated to dryness. The residue is triturated with chloroform to give the desired product as an yellowish while solid (0.46 g). NMR(200 MHz, $D_2O$)δ 3.17(s, N-Me), 4.75(s, $ArCH_2O$), 8.12(dd, 5-pyr H), 8.66(d, 4-pyr H), 9.05(d, 6-pyr H), 9.3(s, 2-pyr H).

Step C. <u>(1R,5R,6S)-1-methyl-2-(N,N-dimethylamino-3-pyridiniummethylthio)-6-(1R-hydroxyethyl)-carbapen-2-em-3-carboxylate</u>

Reacting 4-nitrobenzyl-(1R,5R,6S)-1-methyl-2-diphenylphosphoryloxy-6-(1R-hydroxyethyl)-carbapen-2-em-3-carboxylate with the product of Step B as described in Example 3, Step C one obtains the desired product. NMR(200 NHz, $D_2O$)δ 1.19(d, 6.5 Hz, C-1 $CH_3$), 1.26(d, 7Hz, C-8 $CH_3$), 3.3(m, C-1 H), 3.46(dd, C-6 H), 4.1(m, C-5 H), 4.21(m, C-8 H), 4.25(ABq, $ArCH_2S$), 8.7(dd, 5-pyr H), 8.54(d, 4-pyr H), 9.0(d, 6-pyr H), 9.12(s, 2-pyr H), UV($H_2O$, nm) $\lambda$max 270,295.

EXAMPLE 5

(1R,5R,6S)-1-methyl-2-(1-methyl-4-amino-1,2,4-triazol-5-ium)-methylthio-carbapen-2-em-3-carboxylate

Step A. 1-Methyl-4-amino-1,2,4-triazol-5-ium-carbinol chloride

2-Methyl-3-hydroxymethyl-1,2,4-triazol (0.5 g) is treated with O-(2,4,6-triisopropylbenzenesulfonyl)hydroxylamine (1.5 g) in methylene chloride (10 ml) and the mixture is warmed to 35° for 15 minutes. Aliquat 336 (2.23 ml, 1.1 eq) is added and the reaction mixture is partitioned between methylene chloride and water. The aqueous phase is separated, the organic phase is washed twice with water and the combined aqueous phase is evaporated to dryness to give the desired product (0.65 g).
NMR(200 MHz, $D_2O$)δ 4.08(s, N-Me), 5.4(s, $ArCH_2O$), 8.88(s, Triazol H).

Step B. 1-Methyl-4-amino-5-bromomethyl-1,2,4-triazolium chloride

The product of Step A is treated under the reaction conditions of Example 1, Step B, to give the desired product.
NMR(200 MHz, $D_2O$)δ 4.24(s, N-Me), 5.23(s, $ArCH_2O$), 8.98(s,triazol H).

Step C. 1-Methyl-4-amino-1,2,4-triazol-5-ium-methylthiol chloride

The product of Step B is reacted under the conditions of Example 1, Step C to give the desired compound.
NMR(200 MHz, $D_2O$)δ 4.16(s, N-Me), 4.31(s, $ArCH_2S$), 8.84(s, triazole H).

Step D. <u>(1R,5R,6S)-1-methyl-2-(1-methyl-4-amino-1,2,4-triazol-5-ium)-methylthiocarbapen-2-em-3-carboxylate</u>

Starting with 4-nitrobenzyl-(1R,5R,6S)-1-methyl-2-diphenylphosphoryloxy-6-(1R-hydroxyethyl)-carbapen-2-em-3-carboxylate and treating it with the product of step C under the reaction conditions of Example 1, Step D one obtains the title compound. NMR(200 MHz, $D_2O$)δ 1.25(d, j=7, C-1 Me), 1.32(d, J=6.5, C-8 Me), 3.58(dd, C-6 H), 3.64(m, C-1 H), 4.11(s, N-Me), 4.28(m, C-8 H), 4.32(dd, C-5 H), 4.74(ABq, $CH_2$-S), 8.82(s, triazole H). UV nm $\lambda$max 296, IR $cm^{-1}$ 1765,1585.

## EXAMPLE 6

<u>(5R,6S)-2-(1-methyl-4-amino-1,2,4-triazol-5-ium)-methylthio-carbapen-2-em-3-carboxylate</u>

Starting with 4-nitrobenzyl-(5R,6S)-2-diphenylphosphoryloxy-6-(1R-hydroxyethyl)-carbapen-2-em-3-carboxylate and treating it with the product of Example 5, Step C under the reaction conditions of Example 1, Step D one obtains the title compound. NMR(200 MHz, $D_2O$)d 1.32(d, J=6.5Hz, C-8 Me), 3.21(dd, J=9,16 Hz, C-1 Ha), 3.5(dd, J=9,10 Hz, C-1 Hb), 3.53(dd, C-6 H), 4.14(s, N-Me), 4.29(m, C-5 H and C-8 H), 4.6(ABq, $ArCH_2S$), 8.85(s, triazol H). UV($H_2O$, nm) max 297. IR (nujol) $cm^{-1}$ 1770(b-lactam), 1580(carboxylate).

EXAMPLE 7

(1R,5R,6S)-1-Methyl-2-(N-amino-4-pyridiniummethylthio)-6-(1R-hydroxyethyl)-carbapen-2-em-3-carboxylate

Step A. 4-Pyridylcarbinol mesylate

A solution of 4-pyridylcarbinol (2.15 g, 0.0197 mmol) in methylene chloride (20 ml) is cooled to 0° and treated with triethylamine (1.99 g, 0.0197 mol) followed by methanesulfonyl chloride (2.26 g, 0.0197 mol). The reaction mixture is allowed to warm to room temperature and stirring is continued for 1 hour. The reaction mixture is poured into water, the phases separated and the aqueous phase is extracted 3 times with ethyl acetate. The combined organic phase is washed with water and brine, dried over $MgSO_4$ and evaporated to dryness *in vacuo* to give the mesylate, brick-red solid, mp 59.5-64, NMR(200 MHz, $CDCl_3$)d 3.08(s, $SO_2Me$) 5.27(s, $ArCH_2O$), 7.34(dd, J=1.8, 6.5 Hz, 3 and 5-pyr H), 8.7(dd, J=1.8, J=6.5 Hz, 2 and 6-pyr H). IR(film) $cm^{-1}$ 3030, 2910, 1805, 1355, 1160.

Step B. N-amino-4-methanesulfonyloxymethylpyridinium chloride

To an ice cold solution of 4-Pyridylcarbinol mesylate (585 mg, 3.12 mmol) in dichloromethane (10 ml) is added drop wise a solution of O-(2,4,6-triisopropylbenzenesulfonyl)hydroxylamine (936 mg, 3.12 mmol) in dichloromethane (10 ml). The reaction mixture is allowed to stand at room temperature for 3 hours. Petroleum ether is added and the precipitated tan solid is collected (1.025 g, 67.5%) and dissolved in dichloromethane (10 ml) and water (10 ml) and treated with Aliquat 336 (849 mg, 2.1 mmol). After

stirring for 5 minutes the phases are separated and the aqueous layer is washed with methylene chloride twice and evaporated under reduced pressure to give the desired produce (494 mg, 98.5%). NMR(200 MHz, $D_2O$)d 2.84(s, $SO_2Me$) 5.88(s, $ArCH_2O$), 8.06(dd, J=2.5, 7.0 Hz 3 and 5-pyr H), 8.72(dd, J=2.5, 7.0 Hz, 2 and 6-pyr H). IR(film) $m^{-1}$ 3400-2880, 1640, 1352, 1180.

Step C. (1R,5R,6S)-1-Methyl-2-(N-amino-4-pyridinium-methylthio)-6-(1R-hydroxyethyl)-carbapen-2-em-3-carboxylate

A solution of 4-nitrobenzyl-(1R,5R,6S)-1-methyl-2-diphenylphosphoryloxy-6-(1R-hydroxyethyl)-carbapen-2-em-3-carboxylate (300 mg, 0.504 mmol) in anhydrous DMF (2 ml) is cooled to -40° under nitrogen and treated with DIEA (248 mg, 1.92 mmol followed by dropwise addition of a solution of NaHS (50.8 mg, 0.907 mmol) in DMF (1.5 ml). The reaction is followed by UV. After 1.5 hours N-amino-4-methanesulfonyloxy-methylpyridinium chloride (289 mg, 1.21 mmol) in DMF (2 ml) is added and the reaction mixture is stirred at -40 to -20 for 1.5 hours. The reaction mixture is diluted with n-BuOH (28 ml), ethyl acetate (14 ml), water (28 ml), 0.5 M pH 6.8 N-methylmorpholine hydrochloride buffer (8 ml) and hydrogenatead over 20% Pd(OH) /C (150 mg) for 1.5 hours. The catalyst is filtered off through a pad of celite and washed with water (50 ml). The phases are separated from the filtrate and washings and the aqueous phase is washed with methylene chloride 3 times and with ether twice. The aqueous phase is concentrated under reduced pressure and purified over Dowex resin ($Na^+$

cycle). The eluted fractions containing the desired product are combined and concentrated to 4.5 ml and freeze dried to give the desired product which is further purified by reverse-phase chromatography (ethanol:water 1:4) to give the title compound (21.5 mg, 12%). NMR(200 MHz, $D_2O$)d 1.27(d, J=7.2 Hz, C-1 Me), 1.3(d, J=6.5 Hz, C-8 Me), 3.48(dd, J=2.2, 6.5 Hz, C-6 H), 3.32(m, C-1 H), 4.03-4.28(m, C-5 H, ArCH S), and C-8 H), 7.94(d, J=6.5Hz, 3 and 5-pyrH), 8.45(d, J=6.5Hz, 2 and 4-pyr H). UV($H_2O$, nm) $\lambda$max 300, IR(nujol), $cm^{-1}$ 1750, 1590.

EXAMPLE 8

(5R,6S)-2-(N-amino-3-methyl-2-pyridiniummethyl)-thio-6-(1R-hydroxyethyl)-carbapen-2-em-3-carboxylate

Step A. N-amino-3-methyl-2-pyridiniumcarbinol chloride

Starting with 3-methyl-2-pyridylcarbinol hydrochloride and reacting it with hydroxylamine-O-sulfonic acid as described in Example 1, Step A one obtains the title compound. NMR(200 MHz, $D_2O$)d 2.64(s, $CH_3$-pyr), 5.12(s, $ArCH_2O$), 7.84(dd, 5-pyr H), 8.24(d, 4-pyr H), 8.68(d, 6-pyr H).

Step B. N-amino-3-methyl-2-bromomethylpyridinium chloride

The product of Step A is carried through the reaction conditions of Example 1, Step B to give the title compound. NMR(200 MHz, $D_2O$)d 2.66(s, $CH_3$-pyr), 5.05(s, $ArCH_2Br$), 7.88(dd, 5-pyr H), 8.3(d, 4-pyr H), 8.72(d, 6-pyr H).

Step C. (5R,6S)-2-(N-amino-3-methyl-2-pyridinium-methyl)-thio-6-(1R-hydroxyethyl)-carbapen-2-em-3-carboxylate

Reacting 4-nitrobenzyl-(5R,6S)-2-diphenyl-phosphoryloxy-6-(1R-hydroxyethyl)-carbapen-2-em-3-carboxylate with the product of Step B as described in Example 3, Step C one obtains the desired product. NMR(200 MHz, $D_2O$)δ 1.31(d, 7 Hz, C-8 $CH_3$), 2.64(s, $CH_3$-pyr), 3.38(m, C-1H), 3.5 (dd, C-6H), 4.28(m, C-5H and C-8H), 7.6-8.8(m, ArH). UV nm, λmax 282,sh 300.

## EXAMPLE 9

(1R,5R,6S)-1-methyl-2-(N-amino-3-methyl-2-pyridinium-methylthio)-6-(1R-hydroxyethyl)-carbapen-2-em-3-carboxylate

Reacting 4-nitrobenzyl-(1R,5R,6S)-1-methyl-2-diphenylphosphoryloxy-6-(1R-hydroxyethyl)-carbapen-2-em-3-carboxylate with the product of Example 8, Step B as described in Example 3, Step C one obtains the desired product. NMR(200 MHz, $D_2O$)d 1.29(d, 7 Hz, C-1 $CH_3$), 1.36(d, 6.5 Hz, C-8 $CH_3$), 2.64(s, $CH_3$-pyr), 3.48(dd, J=3.6, C-6 H) 3.6(m, C-1 H), 4.5(m, C-5 H and C-8 H), 4.64(ABq, $ArCH_2S$), 7.8(dd, 5-pyr H), 8.25(d, 4-pyr H), 8.68(d, 6-pyr H). UV nm, λmax 278, sh 300.

## EXAMPLE 10

Utilizing the procedures of Examples 1-9, the following compounds are prepared:

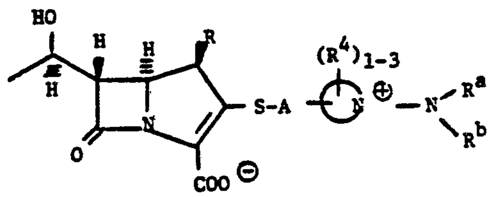

where R is H and alternatively $CH_3$ for each compound defined below

| Compound No. | A | 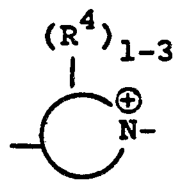 | 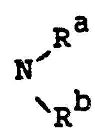 |
|---|---|---|---|
| 1 | $-CH_2-$ | 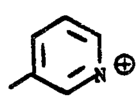 | 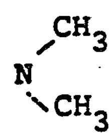 |
| 2 | $-CH_2-$ | 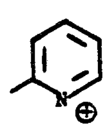 | 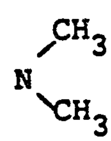 |
| 3 | $-CH(CH_3)-$ | " | $NH_2$ |

| | | | |
|---|---|---|---|
| 4 | " | 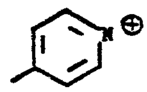 | 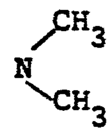 |
| 5 | " | 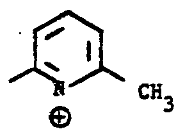 | $NH_2$ |
| 6 | " | 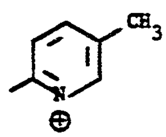 | " |
| 7 | " | 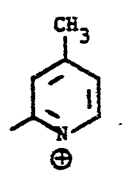 | " |
| 8 | " | 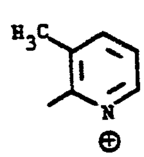 | " |
| 9 | " | 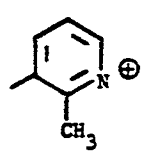 | " |

| | | | |
|---|---|---|---|
| 10 | $-CH_2-$ | 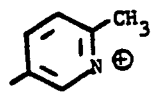 | $NH_2$ |
| 11 | " | 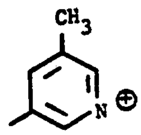 | " |
| 12 | " | 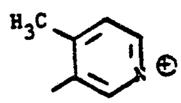 | " |
| 13 | " | 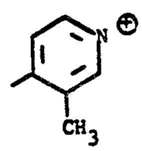 | " |
| 14 | " | 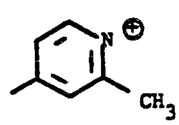 | " |
| 15 | $-CH_2CH_2-$ | 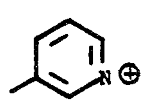 | $NH_2$ |

| | | | |
|---|---|---|---|
| 16 | " | 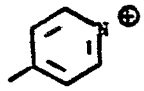 | " |
| 17 | $\begin{matrix}-CH-\\ \mid\\ CH_3\end{matrix}$ | 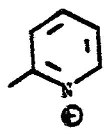 | " |
| 18 | " | 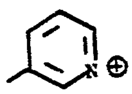 | " |
| 19 | " | 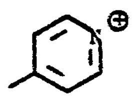 | " |
| 20 | " | 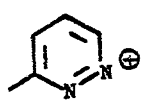 | $NH_2$ |
| 21 | " | 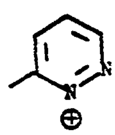 | " |

| | | | |
|---|---|---|---|
| 22 | " | 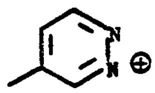 | " |
| 23 | " | 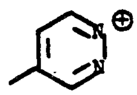 | " |
| 24 | $-CH_2-$ | 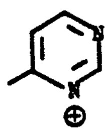 | $NH_2$ |
| 25 | " | 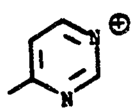 | " |
| 26 | " | 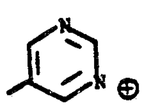 | " |
| 27 | " | 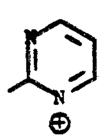 | " |

| | | | |
|---|---|---|---|
| 28 | " | 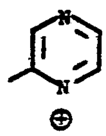 | " |
| 29 | " | 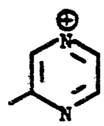 | " |
| 30 | $-CH_2-$ | 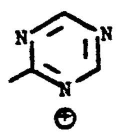 | $NH_2$ |
| 31 | " | 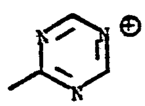 | " |
| 32 | " | 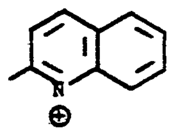 | " |
| 33 | " | 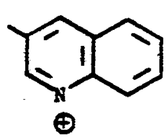 | " |

| | | | |
|---|---|---|---|
| 34 | " | 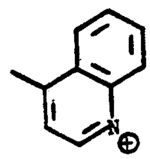 | " |
| 35 | " | 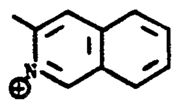 | " |
| 36 | $-CH_2-$ | 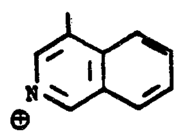 | $NH_2$ |
| 37 | " | 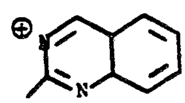 | " |
| 38 | " | 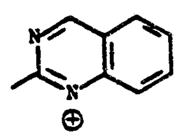 | " |
| 39 | " | 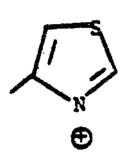 | $NH_2$ |

| | | | |
|---|---|---|---|
| 40 | " | 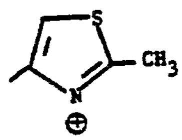 | $NH_2$ |
| 41 | " | 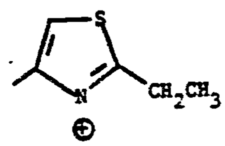 | " |
| 42 | $-CH_2-$ | 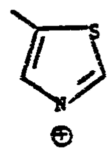 | $NH_2$ |
| 43 | " | 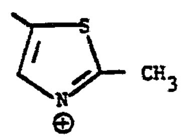 | " |
| 44 | $-\underset{CH_3}{CH}-$ | 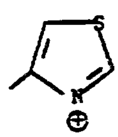 | " |
| 45 | " | 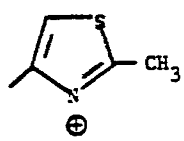 | " |

| | | | |
|---|---|---|---|
| 46 | $-CH_2-$ | 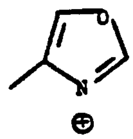 | " |
| 47 | " | " | $N(CH_3)_2$ |
| 48 | " | 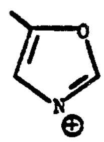 | $NH_2$ |
| 49 | $-CH_2-$ | 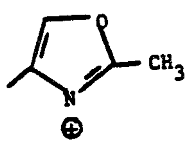 | $NH_2$ |
| 50 | " | 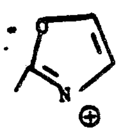 | " |
| 51 | " | 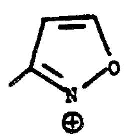 | " |
| 52 | " | 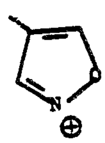 | " |

| | | | |
|---|---|---|---|
| 53 | " | 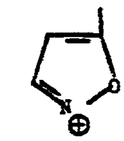 | " |
| 54 | " | 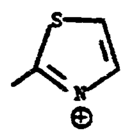 | 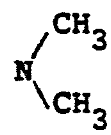 |
| 55 | $-CH_2-$ | 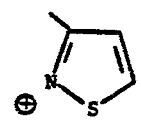 | $NH_2$ |
| 56 | " | 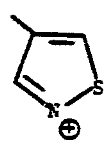 | " |
| 57 | " | 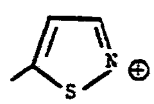 | " |
| 58 | " | 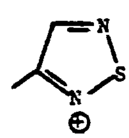 | " |
| 59 | " | 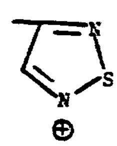 | " |

| | | | |
|---|---|---|---|
| 60 | " | 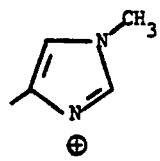 | $NH_2$ |
| 61 | " | " | 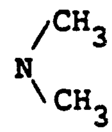 |
| 62 | $-CH_2-$ | 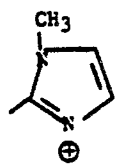 | $NH_2$ |
| 63 | " | 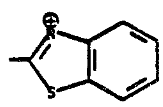 | " |
| 64 | " | 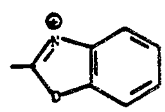 | " |
| 65 | " | 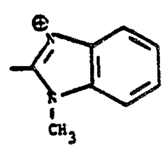 | " |

| | | | |
|---|---|---|---|
| 66 | " | 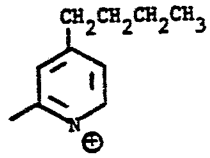 | $NH_2$ |
| 67 | $-CH_2CH_2CH_2-$ | 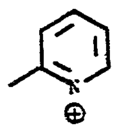 | " |
| 68 | $-CH_2CH(CH_2OH)-$ | 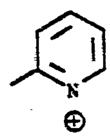 | " |
| 69 | $-CH_2-$ | 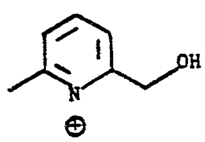 | " |
| 70 | $-CH_2-$ | 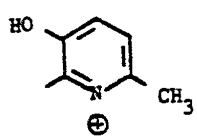 | $NH_2$ |

| | | | |
|---|---|---|---|
| 71 | $-CH_2CH_2CH_2-$ | 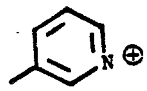 | " |
| 72 | $-CH_2CH_2-$ | 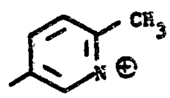 | " |
| 73 | $-CH_2-$ | 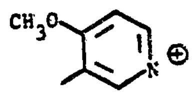 | " |
| 74 | " | 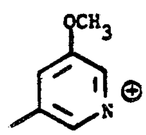 | " |
| 75 | $-CH_2-$ | 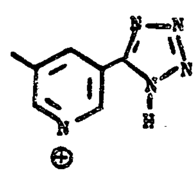 | $NH_2$ |
| 76 | $-CH_2CH_2CH_2-$ | 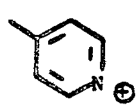 | " |

| | | | |
|---|---|---|---|
| 77 | $CH_2$ | 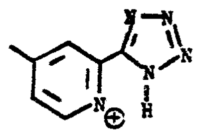 | " |
| 78 | $-CH_2-$ | 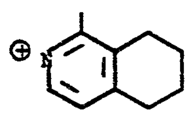 | $NH_2$ |
| 79 | " | 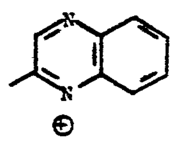 | " |
| 80 | " | 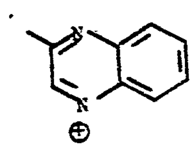 | " |
| 81 | " | 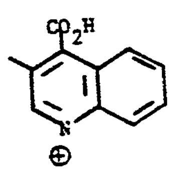 | " |

| | | | |
|---|---|---|---|
| 82 | $-CH_2-$ | 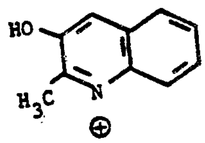 | $NH_2$ |
| 83 | $-CH_2CH_2-$ | 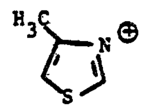 | " |
| 84 | " | 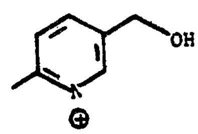 | $NH_2$ |
| 85 | " | 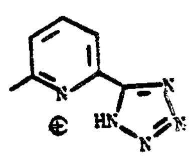 | " |
| 86 | " | 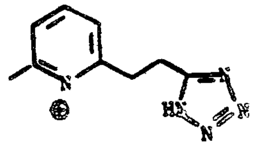 | " |

| | | | |
|---|---|---|---|
| 87 | " | 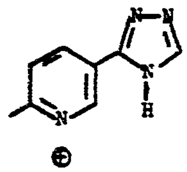 | " |
| 88 | $CH_2$ | 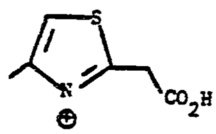 | $NH_2$ |
| 89 | " | 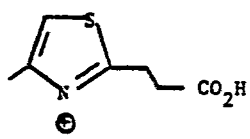 | " |
| 90 | " | 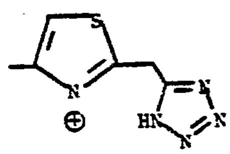 | " |
| 91 | " | 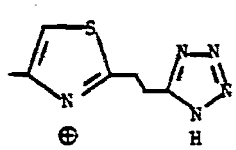 | " |
| 92 | " | 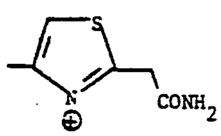 | " |

| | | | |
|---|---|---|---|
| 93 | " | 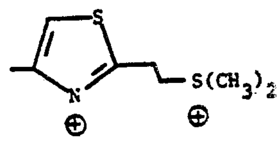 | " |
| 94 | $-CH_2-$ | 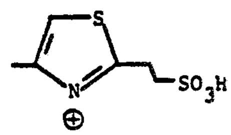 | $NH_2$ |
| 95 | " | 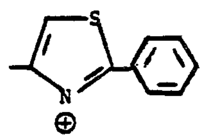 | " |
| 96 | " | 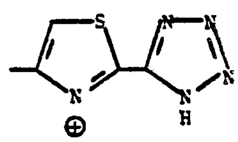 | " |
| 97 | $CH_3$<br>$-CHCH_2-$ | 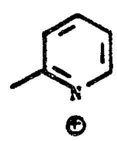 | $NH_2$ |
| 98 | $-CH_2-$ | 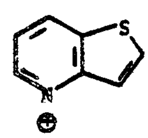 | " |

| | | | |
|---|---|---|---|
| 99 | $-CH_2-$ | 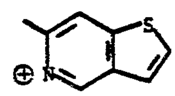 | $NH_2$ |
| 100 | " | 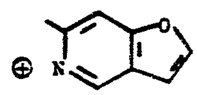 | " |
| 101 | " | 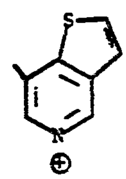 | " |
| 102 | " | 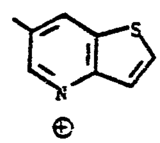 | " |
| 103 | " | 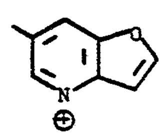 | " |
| 104 | " | 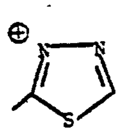 | " |

| | | | |
|---|---|---|---|
| 105 | $-CH_2-$ | 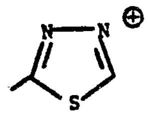 | $NH_2$ |
| 106 | " | 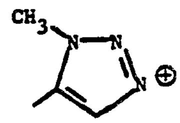 | " |
| 107 | $CH_2$ | 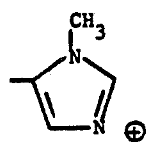 | $NH_2$ |
| 108 | " | 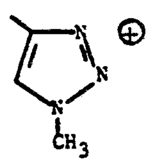 | " |
| 109 | " | 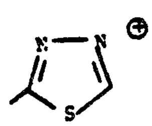 | " |
| 110 | " | 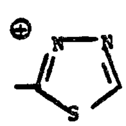 | " |
| 111 | " | 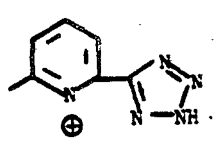 | " |

| | | | |
|---|---|---|---|
| 112 | $-CH_2-$ | 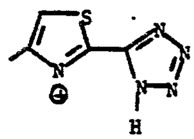 | $NH_2$ |
| 113 | " | 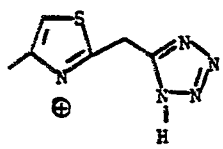 | " |

## EXAMPLE 11

Utilizing the procedures of Examples 1-9, the following compounds are prepared:

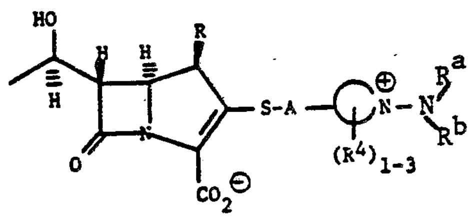

where R is H and alternatively $CH_3$ for each compound defined below

| Compound No. | A | 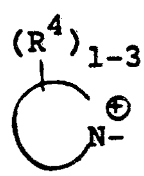 | 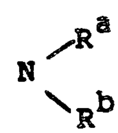 | $R_4$ |
|---|---|---|---|---|
| 1 | $CH_2$ | 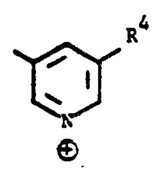 | $NH_2$ | $CO_2H$ |
| 2 | " | " | " | $CONH_2$ |
| 3 | " | " | " | CN |
| 4 | " | " | " | OH |
| 5 | " | " | " | $SO_2NH_2$ |
| 6 | " | " | " | $SO_3H$ |
| 7 | " | " | " | $NMe_2$ |

| | | | | |
|---|---|---|---|---|
| 8 | " | " | " | $CONMe_2$ |
| 9 | " | " | " | $CH_2NMe_2$ |
| 10 | " | " | " | $CH_2CN$ |
| 11 | " | " | " | $CH_2CONH_2$ |
| 12 | " | " | " | $CH_2CO_2H$ |
| 13 | " | " | " | $CH_2SCH_3$ |
| 14 | " | " | " | $CH_2SOCH_3$ |
| 15 | " | " | " | $CH_2SO_2CH_3$ |
| 16 | " | " | " | $SO_2CH_3$ |
| 17 | " | " | " | $SOCH_3$ |
| 18 | " | " | " | 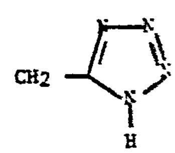 |
| 19 | " | " | " | $CH_2CH_2CO_2H$ |
| 20 | " | " | " | $CH_2SO_3H$ |
| 21 | " | " | " | $CH_2OCH_3$ |
| 22 | " | " | " | 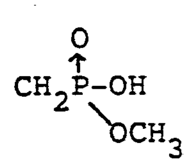 |

| | | | | |
|---|---|---|---|---|
| 23 | " | " | " | $CH_2CH_2SO_3H$ |
| 24 | " | " | " | $CF_3$ |
| 25 | " | " | " | $CH_2O\overset{O}{\overset{\|}{C}}NH_2$ |
| 26 | " | " | " | $CH_2SO_2NH_2$ |
| 27 | " | " | " | Br |
| 28 | " | " | " | Cl |
| 29 | " | " | " | F |
| 30 | " | 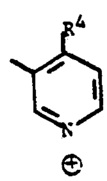 | " | $CO_2H$ |
| 31 | " | " | " | $CONH_2$ |
| 32 | " | " | " | CN |
| 33 | " | " | " | OH |
| 34 | " | " | " | $SONH_2$ |
| 35 | " | " | " | $SO_3H$ |
| 36 | " | " | " | $NMe_2$ |

| | | | | |
|---|---|---|---|---|
| 37 | " | " | " | $CONMe_2$ |
| 38 | " | " | " | $CH_2NMe_2$ |
| 39 | " | " | " | $CH_2CN$ |
| 40 | " | " | " | $CH_2CONH_2$ |
| 41 | " | " | " | $CH_2CO_2H$ |
| 41 | " | " | " | $CH_2SCH_3$ |
| 43 | " | " | " | $CH_2SOCH_3$ |
| 44 | " | " | " | $CH_2SO_2CH_3$ |
| 45 | " | " | " | $SO_2CH_3$ |
| 46 | " | " | " | $SOCH_3$ |
| 47 | " | " | " | 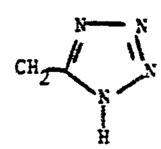 |
| 48 | " | " | " | $CH_2CH_2CO_2H$ |
| 49 | " | " | " | $CH_2SO_3H$ |
| 50 | " | " | " | $CH_2OCH_3$ |
| 51 | " | " | " | 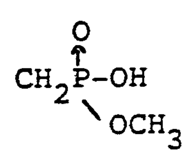 |

| | | | | |
|---|---|---|---|---|
| 52 | " | " | " | $CH_2CH_2SO_3H$ |
| 53 | " | " | " | $CF_3$ |
| 54 | " | " | " | $CH_2O\overset{O}{\overset{\|}{C}}NH_2$ |
| 55 | " | " | " | $CH_2SO_2NH_2$ |
| 56 | " | " | " | $CH_2SO_2NMe_2$ |
| 57 | " | 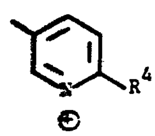 | " | $CO_2H$ |
| 58 | " | " | " | $CONH_2$ |
| 59 | " | " | " | CN |
| 60 | " | " | " | $OCH_3$ |
| 61 | " | " | " | $SO_2NH_2$ |
| 62 | " | " | " | $SO_3H$ |
| 63 | " | " | " | $NMe_2$ |
| 64 | " | " | " | $CONMe_2$ |
| 65 | " | " | " | $CH_2NMe_2$ |

| | | | | |
|---|---|---|---|---|
| 66 | " | " | " | $CH_2CN$ |
| 67 | " | " | " | $CH_2CONH_2$ |
| 68 | " | " | " | $CH_2CO_2H$ |
| 69 | " | " | " | $CH_2SCH_3$ |
| 70 | " | " | " | $CH_2SOCH_3$ |
| 71 | " | " | " | $CH_2SO_2CH_3$ |
| 72 | " | " | " | $SO_2CH_3$ |
| 73 | " | " | " | $SOCH_3$ |
| 74 | " | " | " | 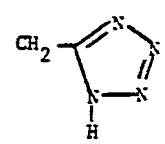 |
| 75 | " | " | " | $CH_2CH_2CO_2H$ |
| 76 | " | " | " | $CH_2SO_3H$ |
| 77 | " | " | " | $CH_2OCH_3$ |
| 78 | " | " | " | 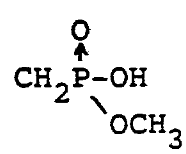 |
| 79 | " | " | " | $CH_2CH_2SO_3H$ |

| | | | | |
|---|---|---|---|---|
| 80 | " | " | " | $CF_3$ |
| 81 | " | " | " | $CH_2O\overset{O}{C}NH_2$ |
| 82 | " | " | " | $CH_2SO_2NH_2$ |
| 83 | " | " | " | $CH_2SO_2NMe_2$ |
| 84 | " | " | " | 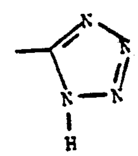 |
| 85 | " | 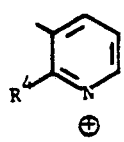 | " | $CO_2H$ |
| 86 | " | " | " | $CONH_2$ |
| 87 | " | " | " | CN |
| 88 | " | " | " | $OCH_3$ |
| 89 | " | " | " | $SO_2NH_2$ |
| 90 | " | " | " | $SO_3H$ |
| 91 | " | " | " | $NMe_2$ |
| 92 | " | " | " | $CONMe_2$ |

| | | | | |
|---|---|---|---|---|
| 93 | " | " | " | $CH_2NMe_2$ |
| 94 | " | " | " | $CH_2CN$ |
| 95 | " | " | " | $CH_2CONH_2$ |
| 96 | " | " | " | $CH_2CO_2H$ |
| 97 | " | " | " | $CH_2SCH_3$ |
| 98 | " | " | " | $CH_2SOCH_3$ |
| 99 | $CH_3$-CH | " | " | $CH_2SO_2CH_3$ |
| 100 | " | " | " | $SO_2CH_3$ |
| 101 | " | " | " | $SOCH_3$ |
| 102 | " | " | " | 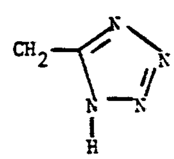 |
| 103 | " | " | " | $CH_2CH_2CO_2H$ |
| 104 | " | " | " | $CH_2SO_3H$ |
| 105 | " | " | " | $CH_2OCH_3$ |
| 106 | " | " | " | 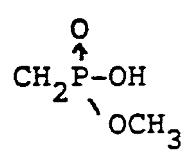 |

| | | | | |
|---|---|---|---|---|
| 107 | " | " | " | $CH_2CH_2SO_3H$ |
| 108 | " | " | " | $CF_3$ |
| 109 | " | " | " | $CH_2OC(=O)NH_2$ |
| 110 | " | " | " | $CH_2SO_2NH_2$ |
| 111 | " | " | " | $CH_2SO_2NMe_2$ |
| 112 | " | " | " | 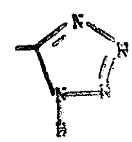 |
| 113 | $-CH_2-$ | 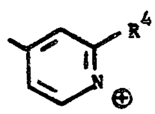 | " | $CO_2H$ |
| 114 | " | " | " | $CONH_2$ |
| 115 | " | " | " | CN |
| 116 | " | " | " | $OCH_3$ |
| 117 | " | " | " | $SO_2NH_2$ |
| 118 | " | " | " | $SO_3H$ |
| 119 | " | " | " | $NMe_2$ |

| | | | | |
|---|---|---|---|---|
| 120 | " | " | " | $CONMe_2$ |
| 121 | " | " | " | $CH_2NMe_2$ |
| 122 | " | " | " | $CH_2CN$ |
| 123 | " | " | " | $CH_2CONH_2$ |
| 124 | " | " | " | $CH_2CO_2H$ |
| 125 | " | " | " | $CH_2SCH_3$ |
| 126 | " | " | " | $CH_2SOCH_3$ |
| 127 | " | " | " | $CH_2SO_2CH_3$ |
| 128 | " | " | " | $SO_2CH_3$ |
| 129 | " | " | " | $SOCH_3$ |
| 130 | " | " | " | 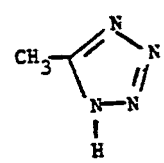 |
| 131 | " | " | " | $CH_2CH_2CO_2H$ |
| 132 | " | " | " | $CH_2SO_3H$ |
| 133 | " | " | " | $CH_2OCH_3$ |
| 134 | " | " | " | 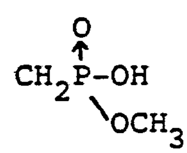 |

| | | | | |
|---|---|---|---|---|
| 135 | " | " | " | $CH_2CH_2SO_3H$ |
| 136 | " | " | " | $CF_3$ |
| 137 | " | " | " | $CH_2O\overset{O}{\overset{\|}{C}}NH_2$ |
| 138 | " | " | " | $CH_2SO_2NH_2$ |
| 139 | " | " | " | $CH_2SO_2NMe_2$ |
| 140 | " | " | " | 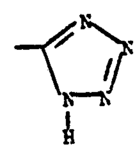 |
| 141 | " | 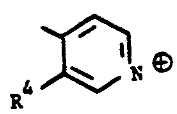 | " | $CO_2H$ |
| 142 | " | " | " | $CONH_2$ |
| 143 | " | " | " | CN |
| 144 | " | " | " | OH |
| 145 | " | " | " | $OCH_3$ |
| 146 | " | " | " | $SO_2NH_2$ |
| 147 | " | " | " | $SO_3H$ |

| | | | | |
|---|---|---|---|---|
| 148 | " | " | " | $NMe_2$ |
| 149 | " | " | " | $CONMe_2$ |
| 150 | " | " | " | $CH_2NMe_2$ |
| 151 | " | " | " | $CH_2CN$ |
| 152 | " | " | " | $CH_2CONH_2$ |
| 153 | " | " | " | $CH_2CO_2H$ |
| 154 | " | " | " | $CH_2SCH_3$ |
| 155 | " | " | " | $CH_2SOCH_3$ |
| 156 | " | " | " | $CH_2SO_2CH_3$ |
| 157 | " | " | " | $SO_2CH_3$ |
| 158 | " | " | " | $SOCH_3$ |
| 159 | " | " | " | 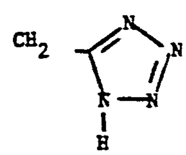 |
| 160 | " | " | " | $CH_2CH_2CO_2H$ |
| 161 | " | " | " | $CH_2SO_3H$ |
| 162 | " | " | " | $CH_2OCH_3$ |

| | | | | |
|---|---|---|---|---|
| 163 | " | " | " | 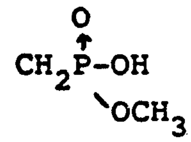 |
| 164 | " | " | " | $CH_2CH_2SO_3H$ |
| 165 | " | " | " | $CF_3$ |
| 166 | " | " | " | 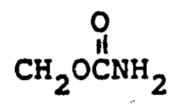 |
| 167 | " | " | " | $CH_2SO_2NH_2$ |
| 168 | " | " | " | $CH_2SO_2NMe_2$ |
| 169 | " | " | " | 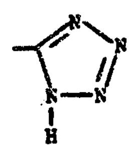 |
| 170 | " | " | " | F |
| 171 | " | " | " | Cl |
| 172 | " | " | " | Br |
| 173 | " | 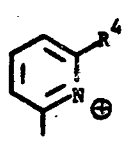 | " | $CO_2H$ |
| 174 | " | " | " | $CONH_2$ |

| | | | | |
|---|---|---|---|---|
| 175 | " | " | " | CN |
| 176 | " | " | " | $SO_2NH_2$ |
| 177 | " | " | " | $SO_3H$ |
| 178 | " | " | " | $NMe_2$ |
| 179 | " | " | " | $CONMe_2$ |
| 180 | " | " | " | $CH_2NMe_2$ |
| 181 | " | " | " | $CH_2CN$ |
| 182 | " | " | " | $CH_2CONH_2$ |
| 183 | " | " | " | $CH_2CO_2H$ |
| 184 | " | " | " | $CH_2SCH_3$ |
| 185 | " | " | " | $CH_2SOCH_3$ |
| 186 | " | " | " | $CH_2SO_2CH_3$ |
| 187 | " | " | " | $SO_2CH_3$ |
| 188 | " | " | " | 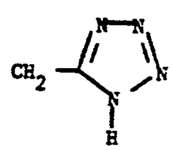 |
| 189 | " | " | " | $CH_2CH_2CO_2H$ |

| | | | | |
|---|---|---|---|---|
| 190 | " | " | " | $CH_2SO_3H$ |
| 191 | " | " | " | $CH_2OCH_3$ |
| 192 | " | " | " | $CH_2\underset{OCH_3}{\overset{O \atop \uparrow}{P}}-OH$ |
| 193 | " | " | " | $CH_2CH_2SO_3H$ |
| 194 | " | " | " | $CF_3$ |
| 195 | " | " | " | $CH_2O\overset{O \atop \|}{C}NH_2$ |
| 196 | " | " | " | $CH_2SO_2NH_2$ |
| 197 | " | 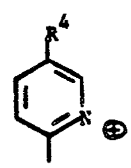 | " | $CO_2H$ |
| 198 | " | " | " | $CONH_2$ |
| 199 | " | " | " | CN |
| 200 | " | " | " | OH |
| 201 | " | " | " | $SO_2NH_2$ |
| 202 | " | " | " | $SO_3H$ |

| | | | | |
|---|---|---|---|---|
| 203 | " | " | " | $NMe_2$ |
| 204 | " | " | " | $CONMe_2$ |
| 205 | " | " | " | $CH_2NMe_2$ |
| 206 | " | " | " | $CH_2CN$ |
| 207 | " | " | " | $CH_2CONH_2$ |
| 208 | " | " | " | $CH_2CO_2H$ |
| 209 | " | " | " | $CH_2SCH_3$ |
| 210 | " | " | " | $CH_2SOCH_3$ |
| 211 | " | " | " | $CH_2SO_2CH_3$ |
| 212 | " | " | " | $SO_2CH_3$ |
| 213 | " | " | " | $SOCH_3$ |
| 214 | " | " | " | 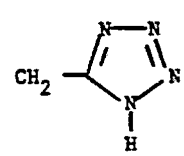 |
| 215 | " | " | " | $CH_2CH_2CO_2H$ |
| 216 | " | " | " | $CH_2SO_3H$ |
| 217 | " | " | " | $CH_2OCH_3$ |

| | | | | |
|---|---|---|---|---|
| 218 | " | " | " | $CH_2P(\rightarrow O)(OH)OCH_3$ |
| 219 | " | " | " | $CH_2CH_2SO_3H$ |
| 220 | " | " | " | $CF_3$ |
| 221 | " | " | " | $CH_2OC(=O)NH_2$ |
| 222 | " | " | " | $CH_2SO_2NH_2$ |
| 223 | " | " | " | Br |
| 224 | " | " | " | Cl |
| 225 | " | " | " | F |
| 226 | " | 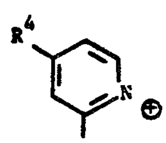 | " | $CO_2H$ |
| 227 | " | " | " | $CONH_2$ |
| 228 | " | " | " | CN |
| 229 | " | " | " | $SO_2NH_2$ |
| 230 | " | " | " | $SO_3H$ |

| | | | | |
|---|---|---|---|---|
| 231 | " | " | " | $NMe_2$ |
| 232 | " | " | " | $CONMe_2$ |
| 233 | " | " | " | $CH_2NMe_2$ |
| 234 | " | " | " | $CH_2CN$ |
| 235 | " | " | " | $CH_2CONH_2$ |
| 236 | " | " | " | $CH_2CO_2H$ |
| 237 | " | " | " | $CH_2SCH_3$ |
| 238 | " | " | " | $CH_2SOCH_3$ |
| 239 | " | " | " | $CH_2SO_2CH_3$ |
| 240 | " | " | " | $SO_2CH_3$ |
| 241 | " | " | " | $SOCH_3$ |
| 242 | " | " | " | 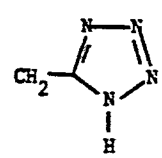 |
| 243 | " | " | " | $CH_2CH_2CO_2H$ |
| 244 | " | " | " | $CH_2SO_3H$ |
| 245 | " | " | " | $CH_2OCH_3$ |

| | | | | |
|---|---|---|---|---|
| 246 | " | " | " | $CH_2\overset{O}{\overset{\uparrow}{P}}\text{-}OH$<br>$\quad\;\, OCH_3$ |
| 247 | " | " | " | $CH_2CH_2SO_3H$ |
| 248 | " | " | " | $CF_3$ |
| 249 | " | " | " | $CH_2O\overset{O}{\overset{\parallel}{C}}NH_2$ |
| 250 | " | " | " | $CH_2SO_2NH_2$ |
| 251 | " | 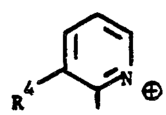 | " | $CO_2H$ |
| 252 | " | " | " | $CONH_2$ |
| 253 | " | " | " | CN |
| 254 | " | " | " | OH |
| 255 | " | " | " | $SO_2NH_2$ |
| 256 | " | " | " | $SO_3H$ |
| 257 | " | " | " | $NMe_2$ |
| 258 | " | " | " | $CONMe_2$ |

| | | | | |
|---|---|---|---|---|
| 259 | " | " | " | $CH_2NMe_2$ |
| 260 | " | " | " | $CH_2CN$ |
| 261 | " | " | " | $CH_2CONH_2$ |
| 262 | " | " | " | $CH_2CO_2H$ |
| 263 | " | " | " | $CH_2SCH_3$ |
| 264 | " | " | " | $CH_2SOCH_3$ |
| 265 | " | " | " | $CH_2SO_2CH_3$ |
| 266 | " | " | " | $SO_2CH_3$ |
| 267 | " | " | " | $SOCH_3$ |
| 268 | " | " | " | 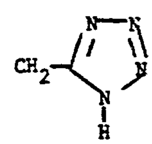 |
| 269 | " | " | " | $CH_2CH_2CO_2H$ |
| 270 | " | " | " | $CH_2SO_3H$ |
| 271 | " | " | " | $CH_2OCH_3$ |
| 272 | " | " | " | $CH_2\underset{OCH_3}{\overset{O\uparrow}{P}}-OH$ |

| | | | | |
|---|---|---|---|---|
| 273 | " | " | " | $CH_2CH_2SO_3H$ |
| 274 | " | " | " | $CF_3$ |
| 275 | " | " | " | $CH_2O\overset{O}{\overset{\|}{C}}NH_2$ |
| 276 | " | " | " | $CH_2SO_2NH_2$ |
| 277 | " | " | " | Br |
| 278 | " | " | " | Cl |
| 279 | " | " | " | F |
| 280 | $CH_2$ | 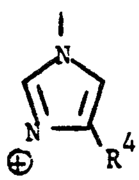 | $NH_2$ | COOH |
| 281 | " | " | " | $CONH_2$ |
| 282 | " | " | " | $CH_2OH$ |
| 283 | " | " | " | $CH_2SO_3H$ |
| 284 | $CH_2$ | 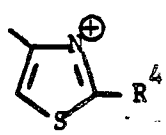 | " | COOH |
| 285 | " | " | " | $CONH_2$ |
| 286 | " | " | " | $CH_2COOH$ |

| | | | | |
|---|---|---|---|---|
| 287 | " | " | " | $CH_2SO_3H$ |
| 288 | " | " | " | $CH_2CN$ |
| 289 | $CH_2$ | 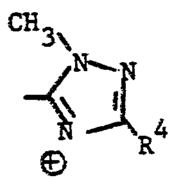 | $NH_2$ | $CH_2SO_3H$ |
| 290 | " | " | " | $CH_2COOH$- |
| 291 | " | " | " | COOH |
| 292 | " | " | " | $CONH_2$ |
| 293 | " | " | " | $CH_2OH$ |

CLAIMS for the contracting states BE, FR, DE, GB, NL, IT, LU, SE, CH

1. A compound of the formula:

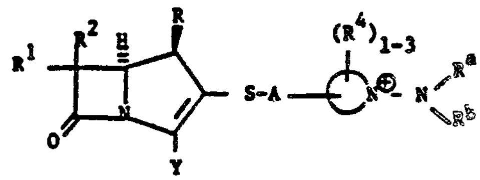

(I.)

wherein:

R is H or $CH_3$;

$R^a$ and $R^b$ are independently hydrogen, $C_1$-$C_4$ straight or branched alkyl, or $C_3$-$C_6$ cycloalkyl;

$R^1$ and $R^2$ are independently H, $CH_3$-, $CH_3CH_2$-, $(CH_3)_2CH$-, $HOCH_2$-, $CH_3CH(OH)$-, $(CH_3)_2C(OH)$_, $FCH_2$-, $F_2CH$-, $F_3C$-, $CH_3CH(F)$-, $CH_3CF_2$-, or $(CH_3)_2C(F)$-;

$R^4$ is independently selected from :

a) a trifluoromethyl group;

b) a halogen atom;

c) an unsubstituted or substituted $C_1$-$C_4$ alkoxy radical;

d) a hydroxy group;

e) an unsubstituted or substituted ($C_1$-$C_6$ alkyl) carbonyloxy radical;

f) a carbamoyloxy radical which is unsubstituted or substituted on nitrogen with one or two $C_1$-$C_4$ alkyl groups;

g) a $C_1$-$C_6$ alkylthio radical, $C_1$-$C_6$ alkylsulfinyl radical or $C_1$-$C_6$ alkylsulfonyl radical, each of which is unsubstituted or substituted on the alkyl group;

h) a sulfamoyl group which is unsubstituted or substituted on nitrogen by one or two $C_1$-$C_4$ alkyl groups;

i) an amino group;

j) a mono ($C_1$-$C_4$ alkyl) amino or di($C_1$-$C_4$ alkyl)amino group, each of which is unsubstituted or substituted on the alkyl group;

k) a formylamino group;

l) an unsubstituted or substituted ($C_1$-$C_6$ alkyl)carbonylamino radical;

m) a ($C_1$-$C_4$ alkoxy) carbonylamino radical;

n) a ureido group in which the terminal nitrogen is unsubstituted or substituted with one or two $C_1$-$C_4$ alkyl groups;

o) a ($C_1$-$C_6$ alkyl)sulfonamido group;

p) a cyano group;

q) a formyl or acetalized formyl radical;

r) an unsubstituted or substituted ($C_1$-$C_6$ alkyl)carbonyl radical wherein the carbonyl is free or acetalized;

s) an unsubstituted or substituted phenylcarbonyl or heteroarlycarbonyl radical;

t) a hydroximinomethyl radical in which the oxygen or carbon atom is optionally substituted by a $C_1$-$C_4$ alkyl group;

u) a ($C_1$-$C_6$ alkoxy)carbonyl radical;

v) a carbamoyl radical which is unsubstituted or substituted on nitrogen by one or two $C_1$-$C_4$ alkyl groups;

w) an N-hydroxycarbamoyl or N($C_1$-$C_4$ alkoxy)carbamoyl radical in which the nitrogen atom may be additionally substituted by a $C_1$-$C_4$ alkyl group;

x) a thiocarbamoyl group;

y) an amidino group 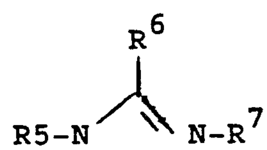

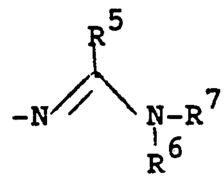

where $R^5$, $R^6$ and $R^7$ are independently hydrogen, $C_1$-$C_4$alkyl or wherein two of the alkyl groups together form a $C_2$-$C_6$alkylidene radical optionally interrupted by a heteroatom and joined together to form a ring;

z) a carboxamidino group 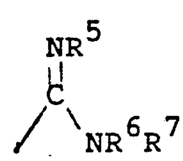, where $R^5$, $R^6$ and $R^7$ are as defined above;

aa) a quanidinyl group where $R^6$ in ab) above is $NR^8R^9$ and $R^8$ and $R^9$ are as defined for $R^5$ through $R^7$ above;

ab) hydrogen;

ac) an unsubstituted or substituted $C_1$-$C_6$ alkyl radical;

ad) an unsubstituted or substituted $C_1$-$C_6$ alkenyl radical;

ae) an unsubstituted or substituted $C_1$-$C_6$ alkynyl radical;

af) a $C_3$-$C_7$ cycloalkyl radical in which the ring is substituted or unsubstituted and one or more atoms may be replaced by a heteroatom;

ag) a $C_3$-$C_7$ cycloalkyl methyl radical in which the ring may be substituted and one or more atoms may be replaced by a heteroatom;

ah) an unsubstituted or substituted $C_5$-$C_7$ cycloalkenyl radical;

ai) an unsubstituted or substituted phenyl or heteroaryl radical;

aj) an unsubstituted or substituted phenyl ($C_1$-$C_4$ alkyl) or heteroaryl ($C_1$-$C_4$ alkyl) radical; and

ak) an acidic side-chain of the structure $-B$ or $-(CH_2)_n-X-(CH_2)_m-W-B$ where:

n is 0-4;

m is 0-4;

X is $CHR^s$, CH=CH, phenylene ($-C_6H_4-$), NH, $N(C_1-C_4$ alkyl), O, S, S=O, C=O, $SO_2$, $SO_2NH$, $CO_2$, CONH, $OCO_2$, OC=O, NHC=O; $R^s$=H, $O(C_1-C_4$ alkyl), $NH_2$, $NH(C_1-C_4$ alkyl), $N(C_1-C_4$ alkyl$)_2$, CN, $CONH_2$, $CON(C_1-C_4$ alkyl$)_2$, $CO_2H$, $SO_2NH_2$, or $SO_2NH(C_1-C_4$ alkyl);

W is a single bond, NH, $N(C_1-C_4$ alkyl), O, or S;

B is an acidic function selected from carboxy ($CO_2H$), phosphono [$P=O(OH)_2$], alkylphosphono {$P=O(OH)-[C(C_1-C_4$ alkyl)]}, alkylphosphinyl [P=O(OH)-($C_1-C_4$ alkyl)], substituted phosphoramido [$P=O(OH)NH(C_1-C_4$ alkyl) and $P=O(CH)NHP^x$], sulfino ($SO_2H$), sulfo ($SO_3H$), 5-tetrazolyl ($CN_4H$), arylsulfonamido ($SO_2NHR^x$) and acylsulfonamides selected from the structures $CONHSO_2(C_1-C_4$ alkyl), $CONHSO_2N(C_1-C_4$alkyl$)_2$, $SO_2NHCO(C_1-C_4)$ alkyl and $SO_2NHCOR^x$, where

$R^x$ is aryl or heteroaryl;

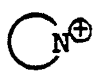 is a mono-or bicyclic quaternary heteroaryl group having 5-11 ring atoms of which, in addition to the quaternary nitrogen, up to four may be heteroatoms independently selected from N, S, or O;

A is a covalent bond or a bridging group selected from $-(CH_2)_{1-4}S-$; $-(CH_2)_{1-4}-O-$; $-(CH_2)_{1-4}-X-(CH_2)_{1-4}$ where X=O, S, NH, or $N(C_1-C_6)$alkyl; substituted or

unsubstituted $C_1$-$C_4$ straight, $C_1$-$C_6$ branched or $C_3$-$C_7$ cycloalkyl groups wherein the substituents are selected from $C_1$-$C_6$ alkyl, O-$C_1$-$C_6$ alkyl, S-$C_1$-$C_6$ alkyl, halo, OH, $CF_3$, CN, $NH_2$, $NHC_1$-$C_6$ alkyl, N($C_1$-$C_6$ alkyl)$_2$, $CO_2H$, $CONH_2$, CONH ($C_1$-$C_6$ alkyl), and CON($C_1$-$C_6$ alkyl)$_2$; and

Y is selected from:
i) COOH or a pharmaceutically acceptable ester or salt thereof,
ii) $COOR^3$ wherein $R^3$ is a readily removable carboxyl protecting group,
iii) COOM wherein M is an alkali metal, or
iv) $COO^{\ominus}$; provided that when Y is other than iv) a counterion $Z^{\ominus}$ is provided.

2. A compound of Claim 1 wherein $R^1/R^2$ is H- and $R^2/R^1$ is $CH_3CH(OH)$-.

3. A compound of Claim 1 wherein the compound is selected from the group consisting of

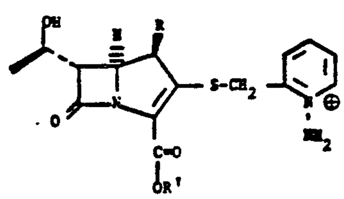

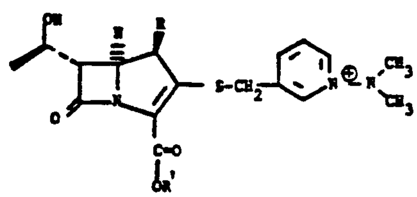

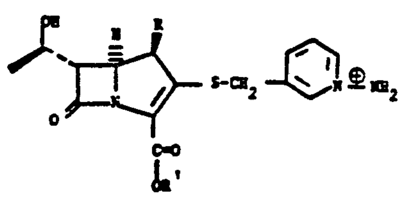

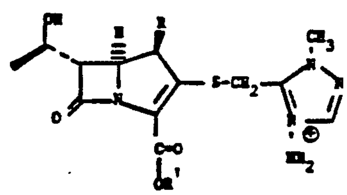

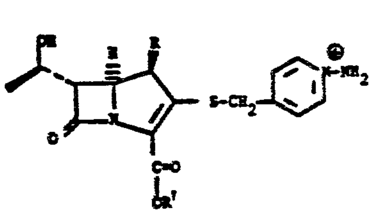
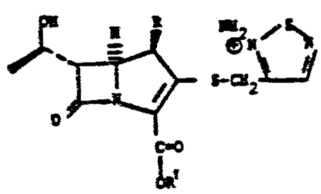
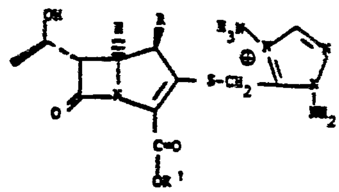

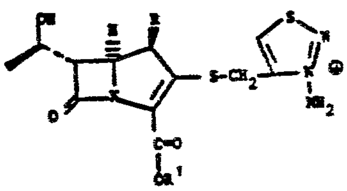
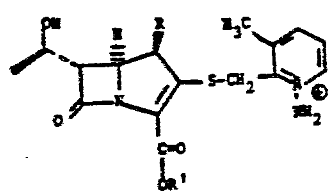
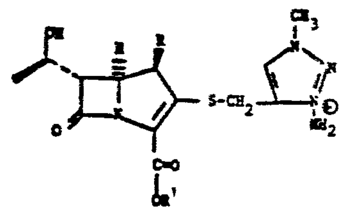

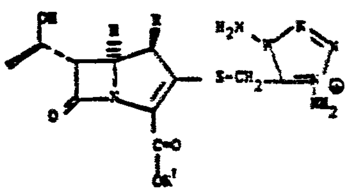

where R = H or $CH_3$;
X = Br, Cl, I or alkyl or aryl-sulfonate; and
R' = H, or a readily removable carboxyl protecting group.

4. A compound of Claim 1 wherein the compound is selected from the group consisting of

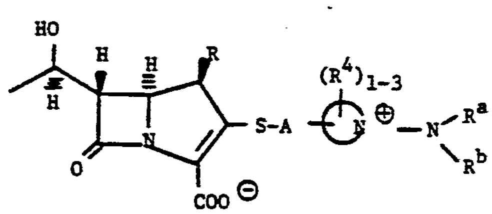

where R is H and alternatively $CH_3$ for each compound defined below

| Compound No. | A | 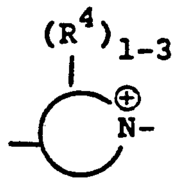 | 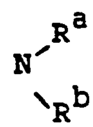 |
| --- | --- | --- | --- |
| 1 | $-CH_2-$ | 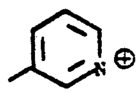 | 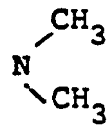 |
| 2 | $-CH_2-$ | 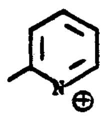 | 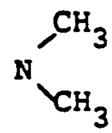 |
| 3 | 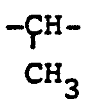 | " | $NH_2$ |
| 4 | " | 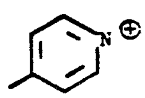 | 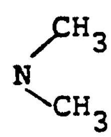 |
| 5 | " | 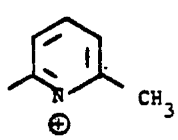 | $NH_2$ |
| 6 | " | 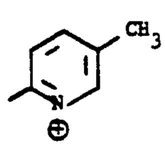 | " |

| | | | |
|---|---|---|---|
| 7 | " | 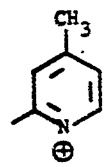 | " |
| 8 | " | 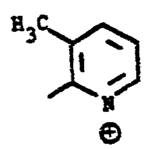 | " |
| 9 | " | 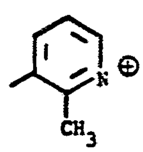 | " |
| 10 | $-CH_2-$ | 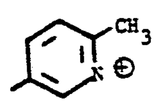 | $NH_2$ |
| 11 | " | 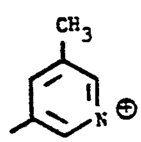 | " |
| 12 | " | 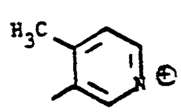 | " |

| | | | |
|---|---|---|---|
| 13 | " | 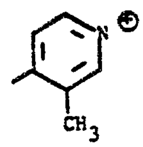 | " |
| 14 | " | 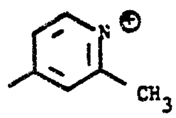 | " |
| 15 | $-CH_2CH_2-$ | 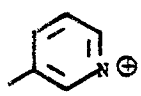 | $NH_2$ |
| 16 | " | 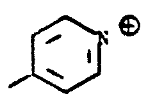 | " |
| 17 | $-\underset{\mid}{CH}-$<br>$CH_3$ | 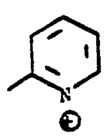 | " |
| 18 | " | 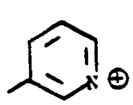 | " |

| | | | |
|---|---|---|---|
| 19 | " | 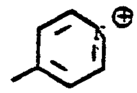 | " |
| 20 | " | 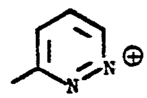 | $NH_2$ |
| 21 | " | 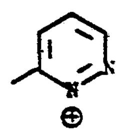 | " |
| 22 | " | 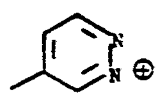 | " |
| 23 | " | 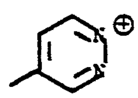 | " |
| 24 | $-CH_2-$ | 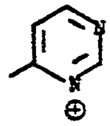 | $NH_2$ |

| | | | |
|---|---|---|---|
| 25 | " | 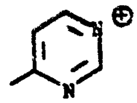 | " |
| 26 | " | 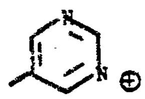 | " |
| 27 | " | 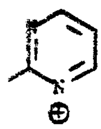 | " |
| 28 | " | 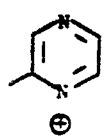 | " |
| 29 | " | 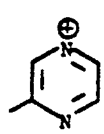 | " |
| 30 | $-CH_2-$ | 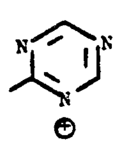 | $NH_2$ |

| | | | |
|---|---|---|---|
| 31 | " | 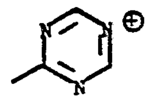 | " |
| 32 | " | 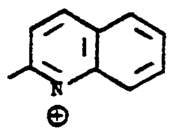 | " |
| 33 | " | 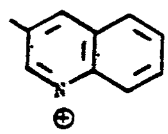 | " |
| 34 | " | 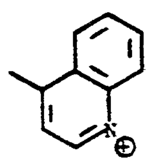 | " |
| 35 | " | 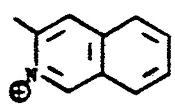 | " |
| 36 | $-CH_2-$ | 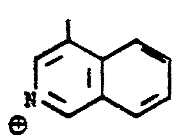 | $NH_2$ |

| | | | |
|---|---|---|---|
| 37 | " | 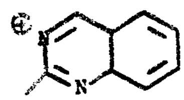 | " |
| 38 | " | 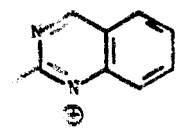 | " |
| 39 | " | 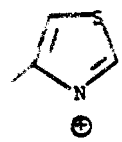 | $NH_2$ |
| 40 | " | 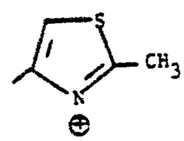 | $NH_2$ |
| 41 | " | 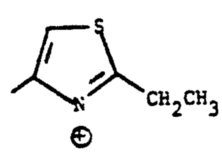 | " |
| 42 | $-CH_2-$ | 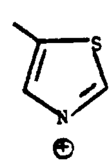 | $NH_2$ |

| | | | |
|---|---|---|---|
| 43 | " | 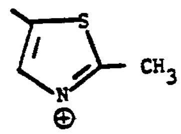 | " |
| 44 | $-CH(CH_3)-$ | 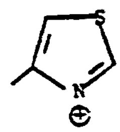 | " |
| 45 | " | 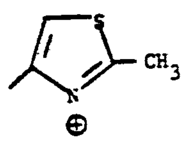 | " |
| 46 | $-CH_2-$ | 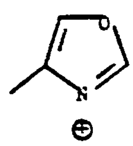 | " |
| 47 | " | " | $N(CH_3)_2$ |
| 48 | " | 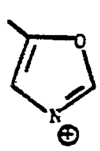 | $NH_2$ |
| 49 | $-CH_2-$ | 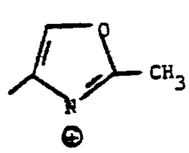 | $NH_2$ |

| | | | |
|---|---|---|---|
| 50 | " | 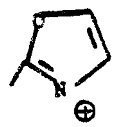 | " |
| 51 | " | 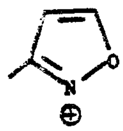 | " |
| 52 | " | 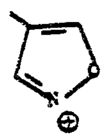 | " |
| 53 | " | 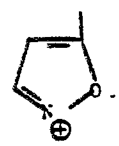 | " |
| 54 | " | 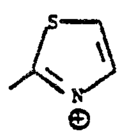 | 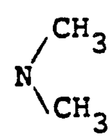 |
| 55 | $-CH_2-$ | 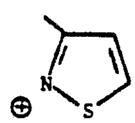 | $NH_2$ |
| 56 | " | 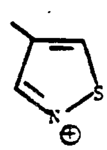 | " |

| | | | |
|---|---|---|---|
| 57 | " | 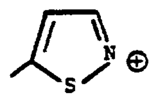 | " |
| 58 | " | 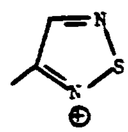 | " |
| 59 | " | 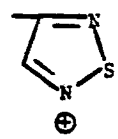 | " |
| 60 | " | 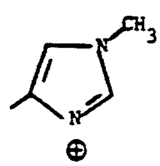 | $NH_2$ |
| 61 | " | " | 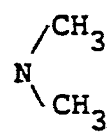 |
| 62 | $-CH_2-$ | 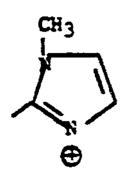 | $NH_2$ |
| 63 | " | 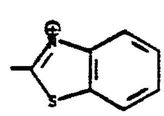 | " |

| | | | |
|---|---|---|---|
| 64 | " | 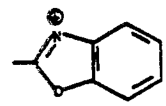 | " |
| 65 | " | 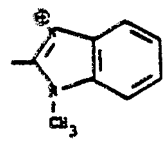 | " |
| 66 | " | 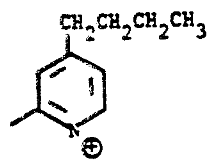 | $NH_2$ |
| 67 | $-CH_2CH_2CH_2-$ | 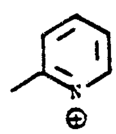 | " |
| 68 | $-CH_2CH-$<br>$CH_2OH$ | 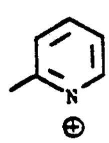 | " |

| | | | |
|---|---|---|---|
| 69 | $-CH_2-$ | 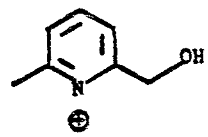 | " |
| 70 | $-CH_2-$ | 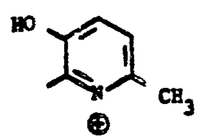 | $NH_2$ |
| 71 | $-CH_2CH_2CH_2-$ | 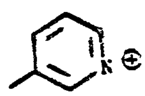 | " |
| 72 | $-CH_2CH_2-$ | 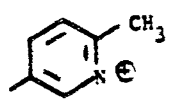 | " |
| 73 | $-CH_2-$ | 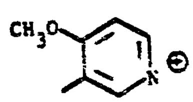 | " |
| 74 | " | 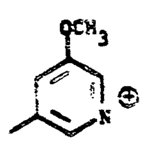 | " |

| | | | |
|---|---|---|---|
| 75 | $-CH_2-$ | 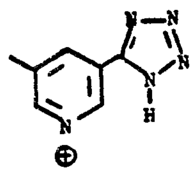 | $NH_2$ |
| 76 | $-CH_2CH_2CH_2-$ | 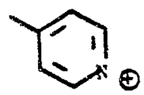 | " |
| 77 | $CH_2$ | 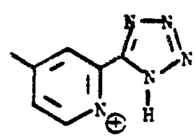 | " |
| 78 | $-CH_2-$ | 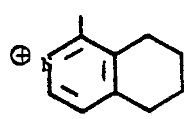 | $NH_2$ |
| 79 | " | 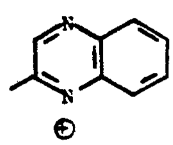 | " |

| | | | |
|---|---|---|---|
| 80 | " | 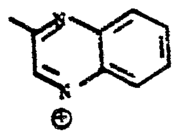 | " |
| 81 | " | 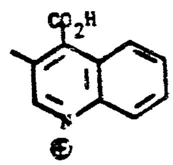 | " |
| 82 | $-CH_2-$ | 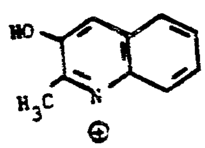 | $NH_2$ |
| 83 | $-CH_2CH_2-$ | 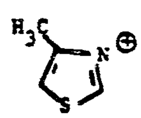 | " |
| 84 | " | 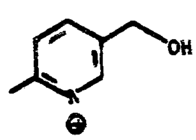 | $NH_2$ |

| | | | |
|---|---|---|---|
| 85 | " | 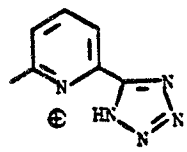 | " |
| 86 | " | 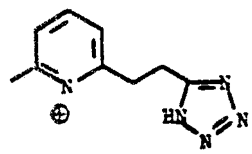 | " |
| 87 | " | 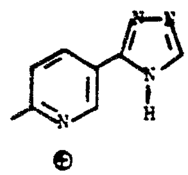 | " |
| 88 | $CH_2$ | 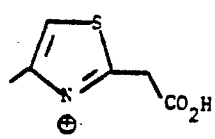 | $NH_2$ |
| 89 | " | 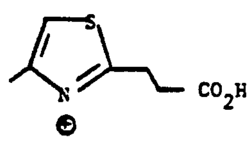 | " |

| | | | |
|---|---|---|---|
| 90 | " | 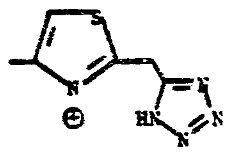 | " |
| 91 | " | 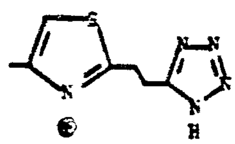 | " |
| 92 | " | 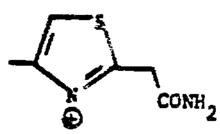 | " |
| 93 | " | 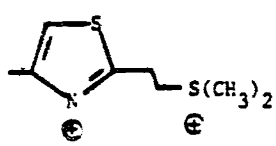 | " |
| 94 | $-CH_2-$ | 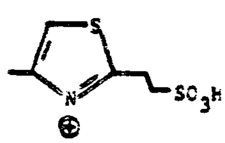 | $NH_2$ |
| 95 | " | 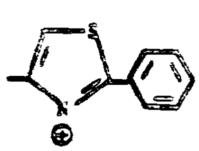 | " |

| | | | |
|---|---|---|---|
| 96 | " | 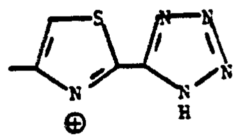 | " |
| 97 | $-\overset{CH_3}{\overset{\vert}{CH}}CH_2-$ | 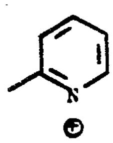 | $NH_2$ |
| 98 | $-CH_2-$ | 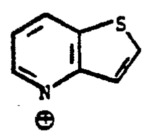 | " |
| 99 | $-CH_2-$ | 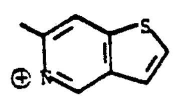 | $NH_2$ |
| 100 | " | 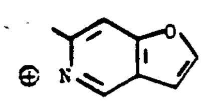 | " |
| 101 | " | 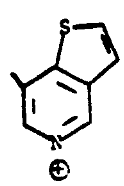 | " |

| | | | |
|---|---|---|---|
| 102 | " | 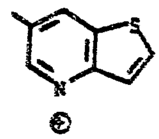 | " |
| 103 | " | 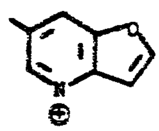 | " |
| 104 | " | 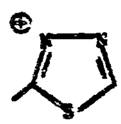 | " |
| 105 | $-CH_2-$ | 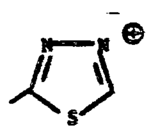 | $NH_2$ |
| 106 | " | 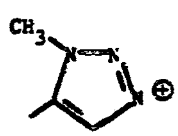 | " |
| 107 | $CH_2$ | 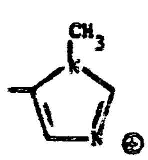 | $NH_2$ |

| | | | |
|---|---|---|---|
| 108 | " | 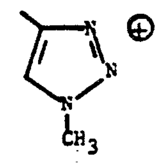 | " |
| 109 | " | 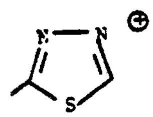 | " |
| 110 | " | 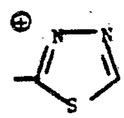 | " |
| 111 | " | 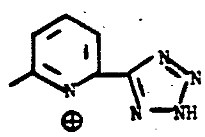 | " |
| 112 | $-CH_2-$ | 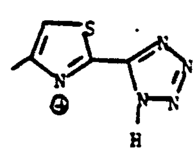 | $NH_2$ |
| 113 | " | 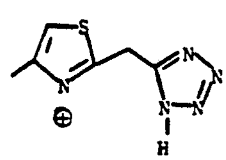 | " |

5. A compound of Claim 1 wherein the compound is selected from the group consisting of

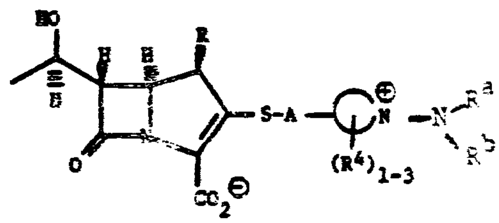

| Compound No. | A | 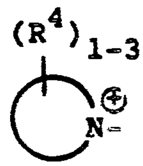 | 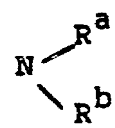 | $R_4$ |
|---|---|---|---|---|
| 1 | $CH_2$ | 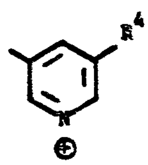 | $NH_2$ | $CO_2H$ |
| 2 | " | " | " | $CONH_2$ |
| 3 | " | " | " | CN |
| 4 | " | " | " | OH |
| 5 | " | " | " | $SO_2NH_2$ |
| 6 | " | " | " | $SO_3H$ |
| 7 | " | " | " | $NMe_2$ |
| 8 | " | " | " | $CONMe_2$ |

| | | | | |
|---|---|---|---|---|
| 9 | " | " | " | $CH_2NMe_2$ |
| 10 | " | " | " | $CH_2CN$ |
| 11 | " | " | " | $CH_2CONH_2$ |
| 12 | " | " | " | $CH_2CO_2H$ |
| 13 | " | " | " | $CH_2SCH_3$ |
| 14 | " | " | " | $CH_2SOCH_3$ |
| 15 | " | " | " | $CH_2SO_2CH_3$ |
| 16 | " | " | " | $SO_2CH_3$ |
| 17 | " | " | " | $SOCH_3$ |
| 18 | " | " | " | 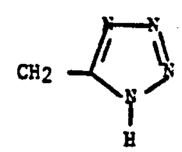 |
| 19 | " | " | " | $CH_2CH_2CO_2H$ |
| 20 | " | " | " | $CH_2SO_3H$ |
| 21 | " | " | " | $CH_2OCH_3$ |
| 22 | " | " | " | 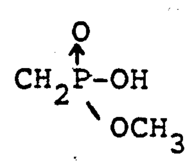 |

| | | | | |
|---|---|---|---|---|
| 23 | " | " | " | $CH_2CH_2SO_3H$ |
| 24 | " | " | " | $CF_3$ |
| 25 | " | " | " | $CH_2O\overset{O}{\overset{\|}{C}}NH_2$ |
| 26 | " | " | " | $CH_2SO_2NH_2$ |
| 27 | " | " | " | Br |
| 28 | " | " | " | Cl |
| 29 | " | " | " | F |
| 30 | " | 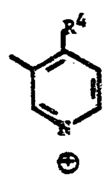 | " | $CO_2H$ |
| 31 | " | " | " | $CONH_2$ |
| 32 | " | " | " | CN |
| 33 | " | " | " | OH |
| 34 | " | " | " | $SONH_2$ |
| 35 | " | " | " | $SO_3H$ |
| 36 | " | " | " | $NMe_2$ |

| | | | | |
|---|---|---|---|---|
| 37 | " | " | " | $CONMe_2$ |
| 38 | " | " | " | $CH_2NMe_2$ |
| 39 | " | " | " | $CH_2CN$ |
| 40 | " | " | " | $CH_2CONH_2$ |
| 41 | " | " | " | $CH_2CO_2H$ |
| 41 | " | " | " | $CH_2SCH_3$ |
| 43 | " | " | " | $CH_2SOCH_3$ |
| 44 | " | " | " | $CH_2SO_2CH_3$ |
| 45 | " | " | " | $SO_2CH_3$ |
| 46 | " | " | " | $SOCH_3$ |
| 47 | " | " | " | 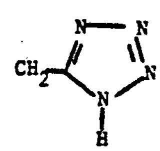 |
| 48 | " | " | " | $CH_2CH_2CO_2H$ |
| 49 | " | " | " | $CH_2SO_3H$ |
| 50 | " | " | " | $CH_2OCH_3$ |
| 51 | " | " | " | 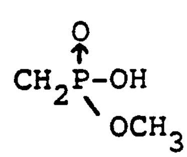 |

| | | | | |
|---|---|---|---|---|
| 52 | " | " | " | $CH_2CH_2SO_3H$ |
| 53 | " | " | " | $CF_3$ |
| 54 | " | " | " | $CH_2OC(=O)NH_2$ |
| 55 | " | " | " | $CH_2SO_2NH_2$ |
| 56 | " | " | " | $CH_2SO_2NMe_2$ |
| 57 | " | 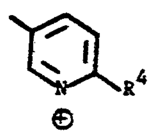 | " | $CO_2H$ |
| 58 | " | " | " | $CONH_2$ |
| 59 | " | " | " | CN |
| 60 | " | " | " | $OCH_3$ |
| 61 | " | " | " | $SO_2NH_2$ |
| 62 | " | " | " | $SO_3H$ |
| 63 | " | " | " | $NMe_2$ |
| 64 | " | " | " | $CONMe_2$ |
| 65 | " | " | " | $CH_2NMe_2$ |

| | | | | |
|---|---|---|---|---|
| 66 | " | " | " | $CH_2CN$ |
| 67 | " | " | " | $CH_2CONH_2$ |
| 68 | " | " | " | $CH_2CO_2H$ |
| 69 | " | " | " | $CH_2SCH_3$ |
| 70 | " | " | " | $CH_2SOCH_3$ |
| 71 | " | " | " | $CH_2SO_2CH_3$ |
| 72 | " | " | " | $SO_2CH_3$ |
| 73 | " | " | " | $SOCH_3$ |
| 74 | " | " | " | 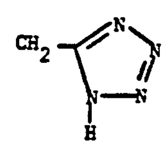 |
| 75 | " | " | " | $CH_2CH_2CO_2H$ |
| 76 | " | " | " | $CH_2SO_3H$ |
| 77 | " | " | " | $CH_2OCH_3$ |
| 78 | " | " | " | 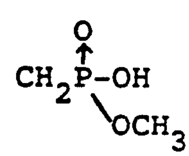 |
| 79 | " | " | " | $CH_2CH_2SO_3H$ |

| | | | | |
|---|---|---|---|---|
| 80 | " | " | " | $CF_3$ |
| 81 | " | " | " | $CH_2OC(=O)NH_2$ |
| 82 | " | " | " | $CH_2SO_2NH_2$ |
| 83 | " | " | " | $CH_2SO_2NMe_2$ |
| 84 | " | " | " | 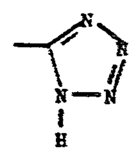 |
| 85 | " | 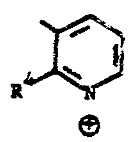 | " | $CO_2H$ |
| 86 | " | " | " | $CONH_2$ |
| 87 | " | " | " | CN |
| 88 | " | " | " | $OCH_3$ |
| 89 | " | " | " | $SO_2NH_2$ |
| 90 | " | " | " | $SO_3H$ |
| 91 | " | " | " | $NMe_2$ |
| 92 | " | " | " | $CONMe_2$ |

| | | | | |
|---|---|---|---|---|
| 93 | " | " | " | $CH_2NMe_2$ |
| 94 | " | " | " | $CH_2CN$ |
| 95 | " | " | " | $CH_2CONH_2$ |
| 96 | " | " | " | $CH_2CO_2H$ |
| 97 | " | " | " | $CH_2SCH_3$ |
| 98 | " | " | " | $CH_2SOCH_3$ |
| 99 | $CH_3$-CH | " | " | $CH_2SO_2CH_3$ |
| 100 | " | " | " | $SO_2CH_3$ |
| 101 | " | " | " | $SOCH_3$ |
| 102 | " | " | " | 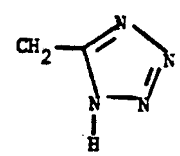 |
| 103 | " | " | " | $CH_2CH_2CO_2H$ |
| 104 | " | " | " | $CH_2SO_3H$ |
| 105 | " | " | " | $CH_2OCH_3$ |
| 106 | " | " | " | 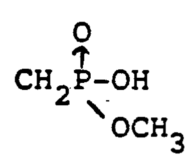 |

| | | | | |
|---|---|---|---|---|
| 107 | " | " | " | $CH_2CH_2SO_3H$ |
| 108 | " | " | " | $CF_3$ |
| 109 | " | " | " | $CH_2O\overset{O}{\overset{\|}{C}}NH_2$ |
| 110 | " | " | " | $CH_2SO_2NH_2$ |
| 111 | " | " | " | $CH_2SO_2NMe_2$ |
| 112 | " | " | " | 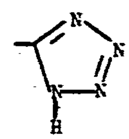 |
| 113 | $-CH_2-$ | 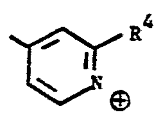 | " | $CO_2H$ |
| 114 | " | " | " | $CONH_2$ |
| 115 | " | " | " | CN |
| 116 | " | " | " | $OCH_3$ |
| 117 | " | " | " | $SO_2NH_2$ |
| 118 | " | " | " | $SO_3H$ |
| 119 | " | " | " | $NMe_2$ |

| | | | | |
|---|---|---|---|---|
| 120 | " | " | " | $CONMe_2$ |
| 121 | " | " | " | $CH_2NMe_2$ |
| 122 | " | " | " | $CH_2CN$ |
| 123 | " | " | " | $CH_2CONH_2$ |
| 124 | " | " | " | $CH_2CO_2H$ |
| 125 | " | " | " | $CH_2SCH_3$ |
| 126 | " | " | " | $CH_2SOCH_3$ |
| 127 | " | " | " | $CH_2SO_2CH_3$ |
| 128 | " | " | " | $SO_2CH_3$ |
| 129 | " | " | " | $SOCH_3$ |
| 130 | " | " | " | 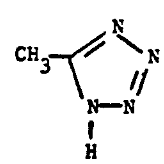 |
| 131 | " | " | " | $CH_2CH_2CO_2H$ |
| 132 | " | " | " | $CH_2SO_3H$ |
| 133 | " | " | " | $CH_2OCH_3$ |
| 134 | " | " | " | 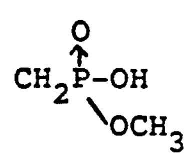 |

| | | | | |
|---|---|---|---|---|
| 135 | " | " | " | $CH_2CH_2SO_3H$ |
| 136 | " | " | " | $CF_3$ |
| 137 | " | " | " | $CH_2O\overset{O}{\overset{\|}{C}}NH_2$ |
| 138 | " | " | " | $CH_2SO_2NH_2$ |
| 139 | " | " | " | $CH_2SO_2NMe_2$ |
| 140 | " | " | " | 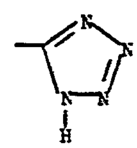 |
| 141 | " | 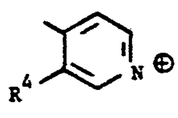 | " | $CO_2H$ |
| 142 | " | " | " | $CONH_2$ |
| 143 | " | " | " | CN |
| 144 | " | " | " | OH |
| 145 | " | " | " | $OCH_3$ |
| 146 | " | " | " | $SO_2NH_2$ |
| 147 | " | " | " | $SO_3H$ |

| | | | | |
|---|---|---|---|---|
| 148 | " | " | " | $NMe_2$ |
| 149 | " | " | " | $CONMe_2$ |
| 150 | " | " | " | $CH_2NMe_2$ |
| 151 | " | " | " | $CH_2CN$ |
| 152 | " | " | " | $CH_2CONH_2$ |
| 153 | " | " | " | $CH_2CO_2H$ |
| 154 | " | " | " | $CH_2SCH_3$ |
| 155 | " | " | " | $CH_2SOCH_3$ |
| 156 | " | " | " | $CH_2SO_2CH_3$ |
| 157 | " | " | " | $SO_2CH_3$ |
| 158 | " | " | " | $SOCH_3$ |
| 159 | " | " | " | 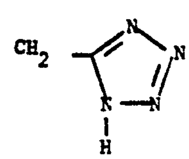 |
| 160 | " | " | " | $CH_2CH_2CO_2H$ |
| 161 | " | " | " | $CH_2SO_3H$ |
| 162 | " | " | " | $CH_2OCH_3$ |

| | | | | |
|---|---|---|---|---|
| 163 | " | " | " | 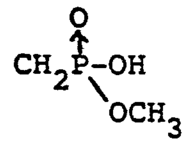 |
| 164 | " | " | " | $CH_2CH_2SO_3H$ |
| 165 | " | " | " | $CF_3$ |
| 166 | " | " | " | 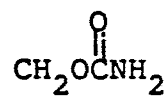 |
| 167 | " | " | " | $CH_2SO_2NH_2$ |
| 168 | " | " | " | $CH_2SO_2NMe_2$ |
| 169 | " | " | " | 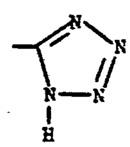 |
| 170 | " | " | " | F |
| 171 | " | " | " | Cl |
| 172 | " | " | " | Br |
| 173 | " | 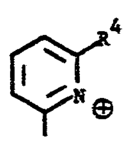 | " | $CO_2H$ |
| 174 | " | " | " | $CONH_2$ |

| | | | | |
|---|---|---|---|---|
| 175 | " | " | " | CN |
| 176 | " | " | " | $SO_2NH_2$ |
| 177 | " | " | " | $SO_3H$ |
| 178 | " | " | " | $NMe_2$ |
| 179 | " | " | " | $CONMe_2$ |
| 180 | " | " | " | $CH_2NMe_2$ |
| 181 | " | " | " | $CH_2CN$ |
| 182 | " | " | " | $CH_2CONH_2$ |
| 183 | " | " | " | $CH_2CO_2H$ |
| 184 | " | " | " | $CH_2SCH_3$ |
| 185 | " | " | " | $CH_2SOCH_3$ |
| 186 | " | " | " | $CH_2SO_2CH_3$ |
| 187 | " | " | " | $SO_2CH_3$ |
| 188 | " | " | " | 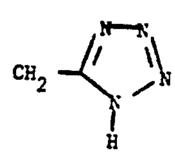 |
| 189 | " | " | " | $CH_2CH_2CO_2H$ |

| | | | | |
|---|---|---|---|---|
| 190 | " | " | " | $CH_2SO_3H$ |
| 191 | " | " | " | $CH_2OCH_3$ |
| 192 | " | " | " | $CH_2\overset{O \atop \uparrow}{\underset{\vert \atop OCH_3}{P}}-OH$ |
| 193 | " | " | " | $CH_2CH_2SO_3H$ |
| 194 | " | " | " | $CF_3$ |
| 195 | " | " | " | $CH_2O\overset{O \atop \Vert}{C}NH_2$ |
| 196 | " | " | " | $CH_2SO_2NH_2$ |
| 197 | " | 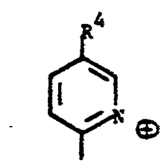 | " | $CO_2H$ |
| 198 | " | " | " | $CONH_2$ |
| 199 | " | " | " | CN |
| 200 | " | " | " | OH |
| 201 | " | " | " | $SO_2NH_2$ |
| 202 | " | " | " | $SO_3H$ |

| | | | | |
|---|---|---|---|---|
| 203 | " | " | " | $NMe_2$ |
| 204 | " | " | " | $CONMe_2$ |
| 205 | " | " | " | $CH_2NMe_2$ |
| 206 | " | " | " | $CH_2CN$ |
| 207 | " | " | " | $CH_2CONH_2$ |
| 208 | " | " | " | $CH_2CO_2H$ |
| 209 | " | " | " | $CH_2SCH_3$ |
| 210 | " | " | " | $CH_2SOCH_3$ |
| 211 | " | " | " | $CH_2SO_2CH_3$ |
| 212 | " | " | " | $SO_2CH_3$ |
| 213 | " | " | " | $SOCH_3$ |
| 214 | " | " | " | 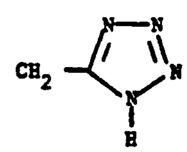 |
| 215 | " | " | " | $CH_2CH_2CO_2H$ |
| 216 | " | " | " | $CH_2SO_3H$ |
| 217 | " | " | " | $CH_2OCH_3$ |

| | | | | |
|---|---|---|---|---|
| 218 | " | " | " | 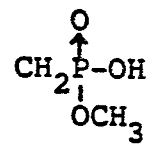 |
| 219 | " | " | " | $CH_2CH_2SO_3H$ |
| 220 | " | " | " | $CF_3$ |
| 221 | " | " | " | 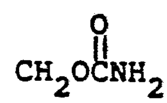 |
| 222 | " | " | " | $CH_2SO_2NH_2$ |
| 223 | " | " | " | Br |
| 224 | " | " | " | Cl |
| 225 | " | " | " | F |
| 226 | " | 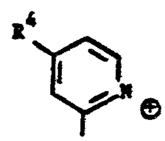 | " | $CO_2H$ |
| 227 | " | " | " | $CONH_2$ |
| 228 | " | " | " | CN |
| 229 | " | " | " | $SO_2NH_2$ |
| 230 | " | " | " | $SO_3H$ |

| | | | | |
|---|---|---|---|---|
| 231 | " | " | " | $NMe_2$ |
| 232 | " | " | " | $CONMe_2$ |
| 233 | " | " | " | $CH_2NMe_2$ |
| 234 | " | " | " | $CH_2CN$ |
| 235 | " | " | " | $CH_2CONH_2$ |
| 236 | " | " | " | $CH_2CO_2H$ |
| 237 | " | " | " | $CH_2SCH_3$ |
| 238 | " | " | " | $CH_2SOCH_3$ |
| 239 | " | " | " | $CH_2SO_2CH_3$ |
| 240 | " | " | " | $SO_2CH_3$ |
| 241 | " | " | " | $SOCH_3$ |
| 242 | " | " | " | 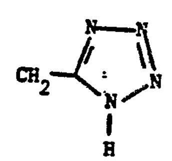 |
| 243 | " | " | " | $CH_2CH_2CO_2H$ |
| 244 | " | " | " | $CH_2SO_3H$ |
| 245 | " | " | " | $CH_2OCH_3$ |

| | | | | |
|---|---|---|---|---|
| 246 | " | " | " | 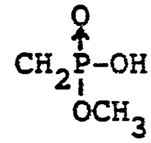 |
| 247 | " | " | " | $CH_2CH_2SO_3H$ |
| 248 | " | " | " | $CF_3$ |
| 249 | " | " | " | 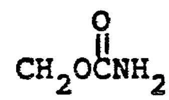 |
| 250 | " | " | " | $CH_2SO_2NH_2$ |
| 251 | " | 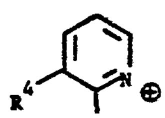 | " | $CO_2H$ |
| 252 | " | " | " | $CONH_2$ |
| 253 | " | " | " | CN |
| 254 | " | " | " | OH |
| 255 | " | " | " | $SO_2NH_2$ |
| 256 | " | " | " | $SO_3H$ |
| 257 | " | " | " | $NMe_2$ |
| 258 | " | " | " | $CONMe_2$ |

| | | | | |
|---|---|---|---|---|
| 259 | " | " | " | $CH_2NMe_2$ |
| 260 | " | " | " | $CH_2CN$ |
| 261 | " | " | " | $CH_2CONH_2$ |
| 262 | " | " | " | $CH_2CO_2H$ |
| 263 | " | " | " | $CH_2SCH_3$ |
| 264 | " | " | " | $CH_2SOCH_3$ |
| 265 | " | " | " | $CH_2SO_2CH_3$ |
| 266 | " | " | " | $SO_2CH_3$ |
| 267 | " | " | " | $SOCH_3$ |
| 268 | " | " | " | 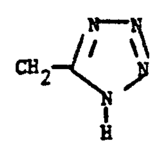 |
| 269 | " | " | " | $CH_2CH_2CO_2H$ |
| 270 | " | " | " | $CH_2SO_3H$ |
| 271 | " | " | " | $CH_2OCH_3$ |
| 272 | " | " | " | 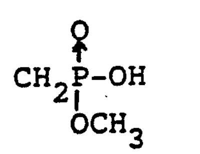 |

| | | | | |
|---|---|---|---|---|
| 273 | " | " | " | $CH_2CH_2SO_3H$ |
| 274 | " | " | " | $CF_3$ |
| 275 | " | " | " | $CH_2OC(=O)NH_2$ |
| 276 | " | " | " | $CH_2SO_2NH_2$ |
| 277 | " | " | " | Br |
| 278 | " | " | " | Cl |
| 279 | " | " | " | F |
| 280 | $CH_2$ | 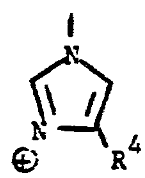 | $NH_2$ | COOH |
| 281 | " | " | " | $CONH_2$ |
| 282 | " | " | " | $CH_2OH$ |
| 283 | " | " | " | $CH_2SO_3H$ |
| 284 | $CH_2$ | 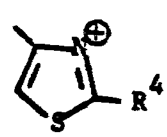 | " | COOH |
| 285 | " | " | " | $CONH_2$ |
| 286 | " | " | " | $CH_2COOH$ |

| | | | | |
|---|---|---|---|---|
| 287 | " | " | " | $CH_2SO_3H$ |
| 288 | " | " | " | $CH_2CN$ |
| 289 | $CH_2$ | 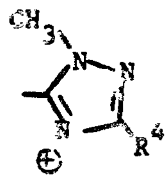 | $NH_2$ | $CH_2SO_3H$ |
| 290 | " | " | " | $CH_2COOH$- |
| 291 | " | " | " | COOH |
| 292 | " | " | " | $CONH_2$ |
| 293 | " | " | " | $CH_2OH$ |

6. A pharmaceutical composition for antibacterial use comprising an antibacterially effective amount of a compound of Claim 1 and a pharmaceutically acceptable carrier.

7. The use of a compound of Claim 1 for the preparation of a medicament useful for the treatment of bacterial infections.

8. The combination of a compound of Claim 1 and a dehydropeptidase inhibitor.

9. A pharmaceutical composition for antibacterial use comprising an antibacterially effective amount of a compound of Claim 1, an inhibitorily effective amount of a dehydropeptidase inhibitor, and, optionally, a pharmaceutically acceptable carrier.

10. The use of a combination of Claim 8 for the preparation of a medicament useful for the treatment of bacterial infections.

CLAIMS for the contracting states AT, GR, ES.

1. A process for the preparation of a compound of the formula:

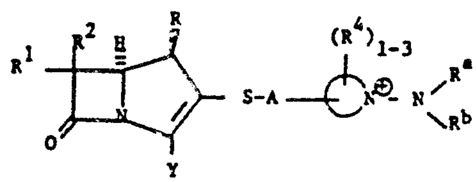

(I.)

wherein:

R is H or $CH_3$;

$R^a$ and $R^b$ are independently hydrogen, $C_1$-$C_4$ straight or branched alkyl, or $C_3$-$C_6$ cycloalkyl;

$R^1$ and $R^2$ are independently H, $CH_3$-, $CH_3CH_2$-, $(CH_3)_2CH$-, $HOCH_2$-, $CH_3CH(OH)$-, $(CH_3)_2C(OH)$-, $FCH_2$-, $F_2CH$-, $F_3C$-, $CH_3CH(F)$-, $CH_3CF_2$-, or $(CH_3)_2C(F)$-;

$R^4$ is independently selected from :

a) a trifluoromethyl group;

b) a halogen atom;

c) an unsubstituted or substituted $C_1$-$C_4$ alkoxy radical;

d) a hydroxy group;

e) an unsubstituted or substituted ($C_1$-$C_6$ alkyl) carbonyloxy radical;

f) a carbamoyloxy radical which is unsubstituted or substituted on nitrogen with one or two $C_1$-$C_4$ alkyl groups;

g) a $C_1$-$C_6$ alkylthio radical, $C_1$-$C_6$ alkylsulfinyl radical or $C_1$-$C_6$ alkylsulfonyl radical, each of which is unsubstituted or substituted on the alkyl group;

h) a sulfamoyl group which is unsubstituted or substituted on nitrogen by one or two $C_1$-$C_4$ alkyl groups;

i) an amino group;

j) a mono ($C_1$-$C_4$ alkyl) amino or di($C_1$-$C_4$ alkyl)amino group, each of which is unsubstituted or substituted on the alkyl group;

k) a formylamino group;

l) an unsubstituted or substituted ($C_1$-$C_6$ alkyl)carbonylamino radical;

m) a ($C_1$-$C_4$ alkoxy) carbonylamino radical;

n) a ureido group in which the terminal nitrogen is unsubstituted or substituted with one or two $C_1$-$C_4$ alkyl groups;

o) a ($C_1$-$C_6$ alkyl)sulfonamido group;

p) a cyano group;

q) a formyl or acetalized formyl radical;

r) an unsubstituted or substituted ($C_1$-$C_6$ alkyl)carbonyl radical wherein the carbonyl is free or acetalized;

s) an unsubstituted or substituted phenylcarbonyl or heteroarlycarbonyl radical;

t) a hydroximinomethyl radical in which the oxygen or carbon atom is optionally substituted by a $C_1$-$C_4$ alkyl group;

u) a ($C_1$-$C_6$ alkoxy)carbonyl radical;

v) a carbamoyl radical which is unsubstituted or substituted on nitrogen by one or two $C_1$-$C_4$ alkyl groups;

w) an N-hydroxycarbamoyl or N($C_1$-$C_4$ alkoxy)carbamoyl radical in which the nitrogen atom may be additionally substituted by a $C_1$-$C_4$ alkyl group;

x) a thiocarbamoyl group;

y) an amidino group 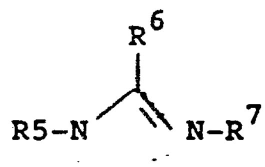

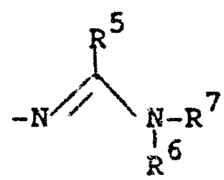

where $R^5$, $R^6$ and $R^7$ are independently hydrogen, $C_1$-$C_4$alkyl or wherein two of the alkyl groups together form a $C_2$-$C_6$alkylidene radical optionally interrupted by a heteroatom and joined together to form a ring;

z) a carboxamidino group 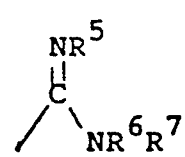, where $R^5$, $R^6$ and $R^7$ are as defined above;

aa) a quanidinyl group where $R^6$ in ab) above is $NR^8R^9$ and $R^8$ and $R^9$ are as defined for $R^5$ through $R^7$ above;

ab) hydrogen;

ac) an unsubstituted or substituted $C_1$-$C_6$ alkyl radical;

ad) an unsubstituted or substituted $C_1$-$C_6$ alkenyl radical;

ae) an unsubstituted or substituted $C_1$-$C_6$ alkynyl radical;

af) a $C_3$-$C_7$ cycloalkyl radical in which the ring is substituted or unsubstituted and one or more atoms may be replaced by a heteroatom;

ag) a $C_3$-$C_7$ cycloalkyl methyl radical in which the ring may be substituted and one or more atoms may be replaced by a heteroatom;

ah) an unsubstituted or substituted $C_5$-$C_7$ cycloalkenyl radical;

ai) an unsubstituted or substituted phenyl or heteroaryl radical;

aj) an unsubstituted or substituted phenyl ($C_1$-$C_4$ alkyl) or heteroaryl ($C_1$-$C_4$ alkyl) radical; and

ak) an acidic side-chain of the structure $-B$ or $-(CH_2)_n-X-(CH_2)_m-W-B$ where:

$n$ is 0-4;

$m$ is 0-4;

X is $CHR^s$, CH=CH, phenylene $(-C_6H_4-)$, NH, $N(C_1-C_4$ alkyl), O, S, S=O, C=O, $SO_2$, $SO_2NH$, $CO_2$, CONH, $OCO_2$, OC=O, NHC=O; $R^s$=H, $O(C_1-C_4$ alkyl), $NH_2$, $NH(C_1-C_4$ alkyl), $N(C_1-C_4$ alkyl$)_2$, CN, $CONH_2$, $CON(C_1-C_4$ alkyl$)_2$, $CO_2H$, $SO_2NH_2$, or $SO_2NH(C_1-C_4$ alkyl);

W is a single bond, NH, $N(C_1-C_4$ alkyl), O, or S;

B is an acidic function selected from carboxy $(CO_2H)$, phosphono $[P=O(OH)_2]$, alkylphosphono $\{P=O(OH)-[C(C_1-C_4$ alkyl)]$\}$, alkylphosphinyl [P=O(OH)-$(C_1-C_4$ alkyl)], substituted phosphoramido $[P=O(OH)NH(C_1-C_4$ alkyl) and $P=O(CH)NHP^x]$, sulfino $(SO_2H)$, sulfo $(SO_3H)$, 5-tetrazolyl $(CN_4H)$, arylsulfonamido $(SO_2NHR^x)$ and acylsulfonamides selected from the structures $CONHSO_2(C_1-C_4$ alkyl), $CONHSO_2N(C_1-C_4$alkyl$)_2$, $SO_2NHCO(C_1-C_4)$ alkyl and $SO_2NHCOR^x$, where

$R^x$ is aryl or heteroaryl;

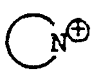 is a mono-or bicyclic quaternary heteroaryl group having 5-11 ring atoms of which, in addition to the quaternary nitrogen, up to four may be heteroatoms independently selected from N, S, or O;

A is a covalent bond or a bridging group selected from $-(CH_2)_{1-4}S-$; $-(CH_2)_{1-4}-O-$; $-(CH_2)_{1-4}-X-(CH_2)_{1-4}$ where X=O, S, NH, or $N(C_1-C_6)$alkyl; substituted or

unsubstituted $C_1$-$C_4$ straight, $C_1$-$C_6$ branched or $C_3$-$C_7$ cycloalkyl groups wherein the substituents are selected from $C_1$-$C_6$ alkyl, O-$C_1$-$C_6$ alkyl, S-$C_1$-$C_6$ alkyl, halo, OH, $CF_3$, CN, $NH_2$, NH$C_1$-$C_6$ alkyl, N($C_1$-$C_6$ alkyl)$_2$, $CO_2H$, $CONH_2$, CONH ($C_1$-$C_6$ alkyl), and CON($C_1$-$C_6$ alkyl)$_2$; and

Y is selected from:

i) COOH or a pharmaceutically acceptable ester or salt thereof,

ii) $COOR^3$ wherein $R^3$ is a readily removable carboxyl protecting group,

iii) COOM wherein M is an alkali metal, or

iv) $COO^{\ominus}$; provided that when Y is other than iv) a counterion $Z^{\ominus}$ is provided,

which process comprises:

(a) the reaction of a compound of formula D - Y with a compound of formula E - SH wherein Y is a leaving group displaceable by a thiol, one of D and E represents a carbapenem nucleus of formula (A) and the other represents a side-chain group of formula (B):

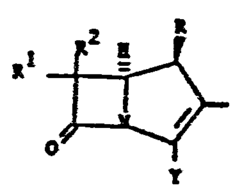

(A)

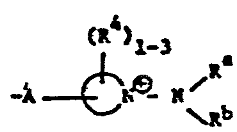

(B)

or (b) reacting a compound of formula:

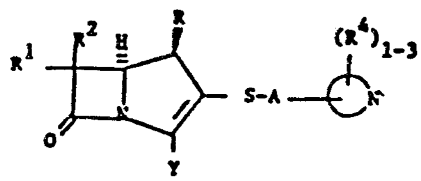

with a compound which provides the N-quaternizing substituent $-NR^aR^b$.

2. A process of Claim 1 wherein $R^1/R^2$ is H- and $R^2/R^1$ is $CH_3CH(OH)-$.

3. A process of Claim 1 wherein the compound is selected from the group consisting of

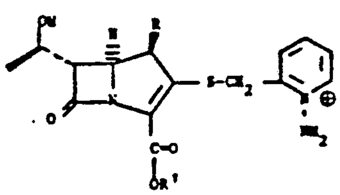

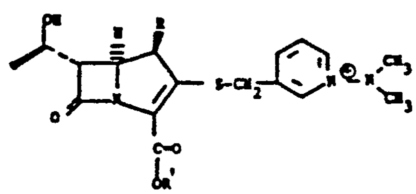

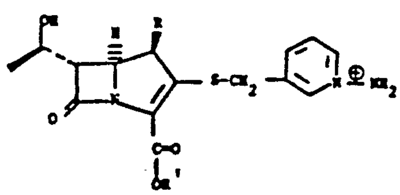

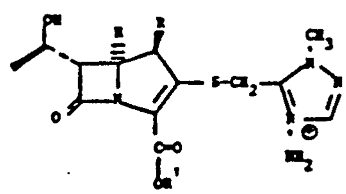

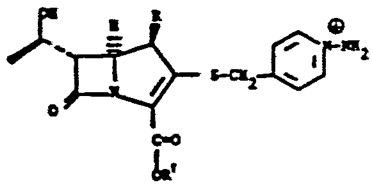 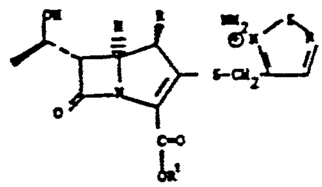 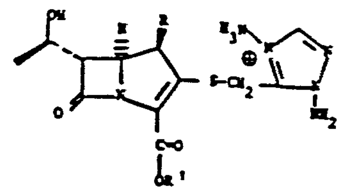

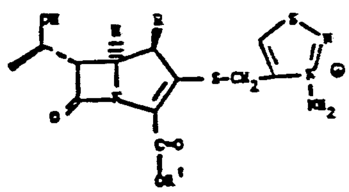 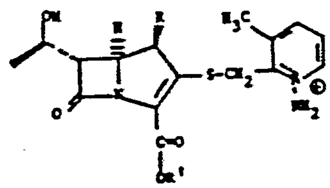 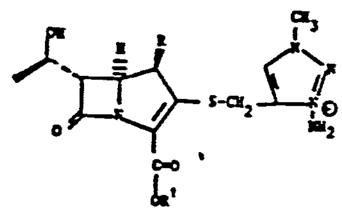

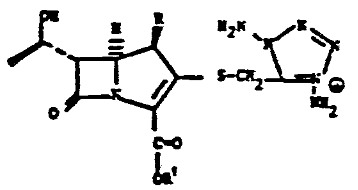

where R = H or $CH_3$;
X = Br, Cl, I or alkyl or aryl-sulfonate; and
R' = H, or a readily removable carboxyl protecting group.

4. A process of Claim 1 wherein the compound is selected from the group consisting of

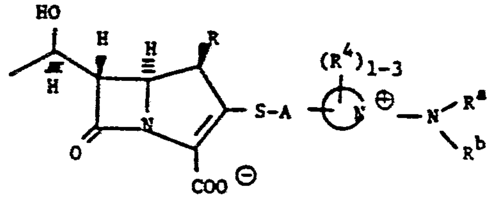

where R is H and alternatively $CH_3$ for each compound defined below

| Compound No. | A | 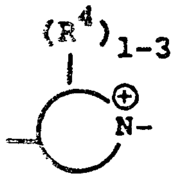 | 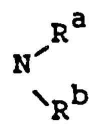 |
| --- | --- | --- | --- |
| 1 | $-CH_2-$ | 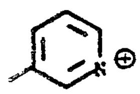 | 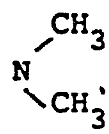 |
| 2 | $-CH_2-$ | 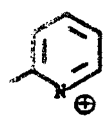 | 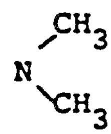 |
| 3 | 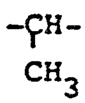 | " | $NH_2$ |
| 4 | " | 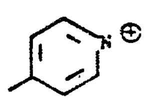 | 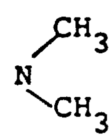 |
| 5 | " | 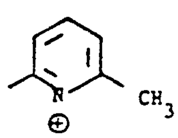 | $NH_2$ |
| 6 | " | 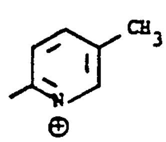 | " |

| | | | |
|---|---|---|---|
| 7 | " | 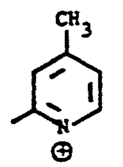 | " |
| 8 | " | 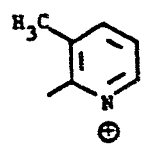 | " |
| 9 | " | 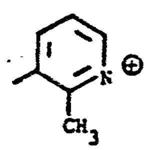 | " |
| 10 | $-CH_2-$ | 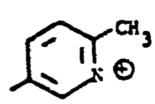 | $NH_2$ |
| 11 | " | 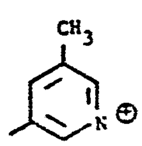 | " |
| 12 | " | 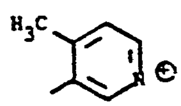 | " |

| | | | |
|---|---|---|---|
| 13 | " | 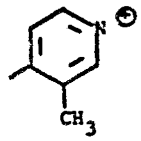 | " |
| 14 | " | 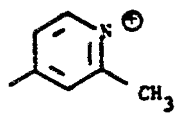 | " |
| 15 | $-CH_2CH_2-$ | 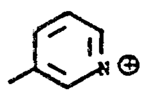 | $NH_2$ |
| 16 | " | 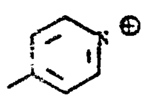 | " |
| 17 | -CH-<br>$CH_3$ | 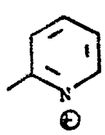 | " |
| 18 | " | 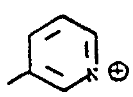 | " |

| | | | |
|---|---|---|---|
| 19 | " | 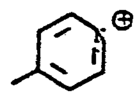 | " |
| 20 | " | 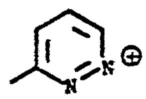 | $NH_2$, |
| 21 | " | 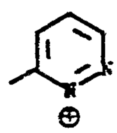 | " |
| 22 | " | 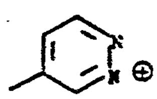 | " |
| 23 | " | 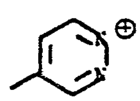 | " |
| 24 | $-CH_2-$ | 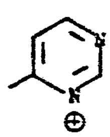 | $NH_2$ |

| | | | |
|---|---|---|---|
| 25 | " | 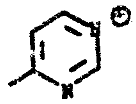 | " |
| 26 | " | 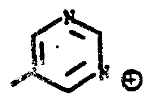 | " |
| 27 | " | 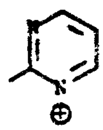 | " |
| 28 | " | 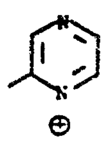 | " |
| 29 | " | 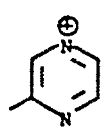 | " |
| 30 | $-CH_2-$ | 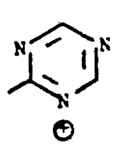 | $NH_2$ |

| | | | |
|---|---|---|---|
| 31 | " | 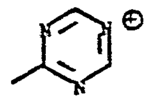 | " |
| 32 | " | 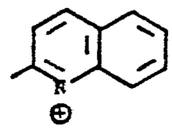 | " |
| 33 | " | 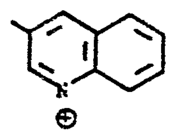 | " |
| 34 | " | 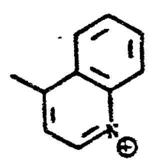 | " |
| 35 | " | 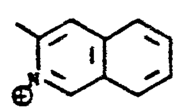 | " |
| 36 | $-CH_2-$ | 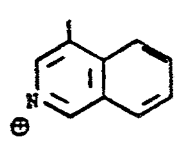 | $NH_2$ |

| | | | |
|---|---|---|---|
| 37 | " | 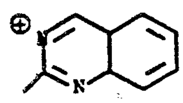 | " |
| 38 | " | 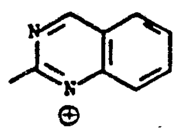 | " |
| 39 | " | 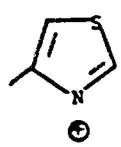 | $NH_2$ |
| 40 | " | 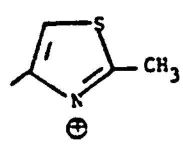 | $NH_2$ |
| 41 | " | 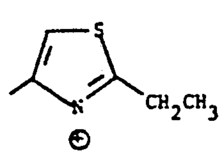 | " |
| 42 | $-CH_2-$ | 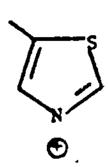 | $NH_2$ |

| | | | |
|---|---|---|---|
| 43 | " | 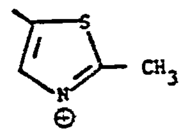 | " |
| 44 | -CH-<br>$CH_3$ | 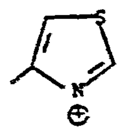 | " |
| 45 | " | 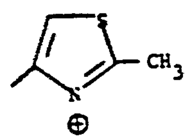 | " |
| 46 | $-CH_2-$ | 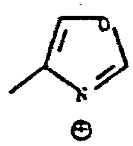 | " |
| 47 | " | " | $CH_3$<br>N<br>$CH_3$ |
| 48 | " | 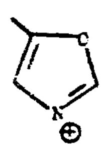 | $NH_2$ |
| 49 | $-CH_2-$ | 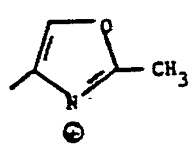 | $NH_2$ |

| | | | |
|---|---|---|---|
| 50 | " | 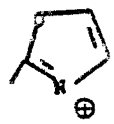 | " |
| 51 | " | 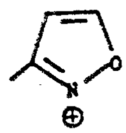 | " |
| 52 | " | 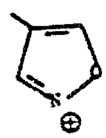 | " |
| 53 | " | 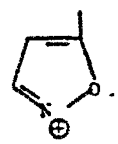 | " |
| 54 | " | 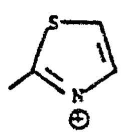 | 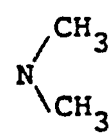 |
| 55 | $-CH_2-$ | 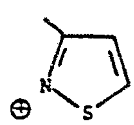 | $NH_2$ |
| 56 | " | 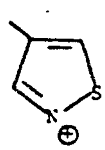 | " |

| | | | |
|---|---|---|---|
| 57 | " | 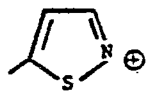 | " |
| 58 | " | 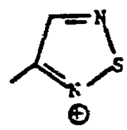 | " |
| 59 | " | 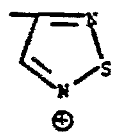 | " |
| 60 | " | 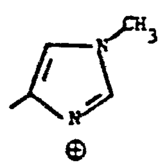 | $NH_2$ |
| 61 | " | " | $N(CH_3)_2$ |
| 62 | $-CH_2-$ | 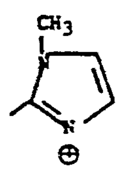 | $NH_2$ |
| 63 | " | 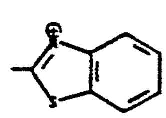 | " |

| | | | |
|---|---|---|---|
| 64 | " | 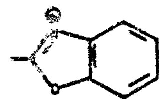 | " |
| 65 | " | 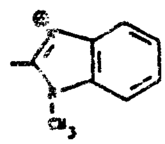 | " |
| 66 | " | 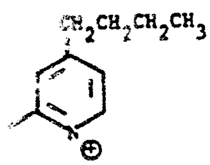 | $NH_2$ |
| 67 | $-CH_2CH_2CH_2-$ | 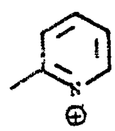 | " |
| 68 | $-CH_2CH-$<br>$CH_2OH$ | 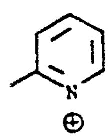 | " |

| | | | |
|---|---|---|---|
| 69 | $-CH_2-$ | 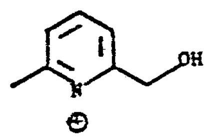 | " |
| 70 | $-CH_2-$ | 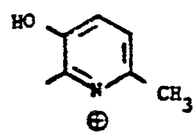 | $NH_2$ |
| 71 | $-CH_2CH_2CH_2-$ | 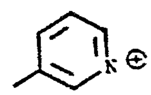 | " |
| 72 | $-CH_2CH_2-$ | 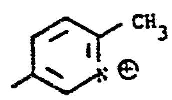 | " |
| 73 | $-CH_2-$ | 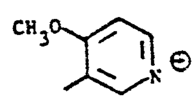 | " |
| 74 | " | 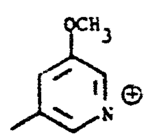 | " |

| | | | |
|---|---|---|---|
| 75 | $-CH_2-$ | 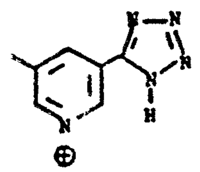 | $NH_2$ |
| 76 | $-CH_2CH_2CH_2-$ | 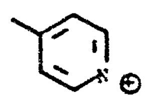 | " |
| 77 | $CH_2$ | 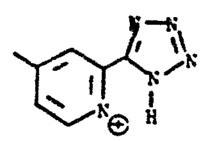 | " |
| 78 | $-CH_2-$ | 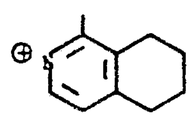 | $NH_2$ |
| 79 | " | 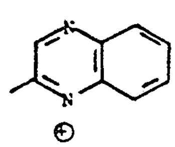 | " |

| | | | |
|---|---|---|---|
| 80 | " | 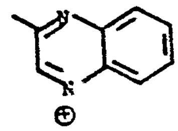 | " |
| 81 | " | 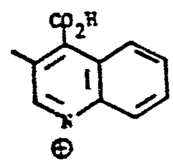 | " |
| 82 | $-CH_2-$ | 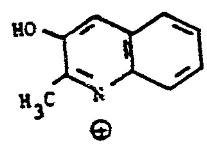 | $NH_2$ |
| 83 | $-CH_2CH_2-$ | 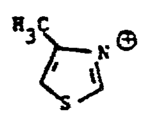 | " |
| 84 | " | 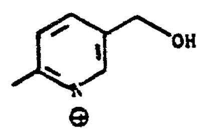 | $NH_2$ |

| | | | |
|---|---|---|---|
| 85 | " | 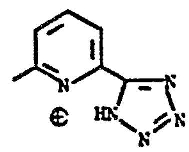 | " |
| 86 | " | 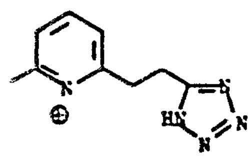 | " |
| 87 | " | 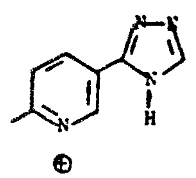 | " |
| 88 | $CH_2$ | 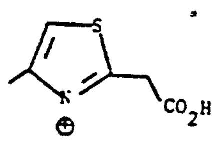 | $NH_2$ |
| 89 | " | 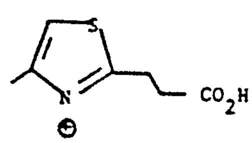 | " |

| | | | |
|---|---|---|---|
| 90 | " | 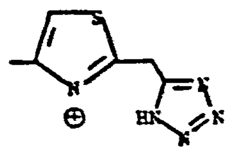 | " |
| 91 | " | 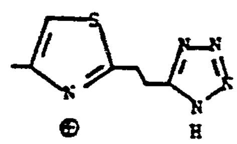 | " |
| 92 | " | 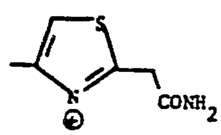 | " |
| 93 | " | 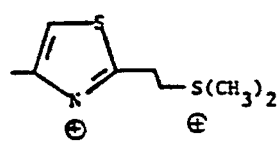 | " |
| 94 | $-CH_2-$ | 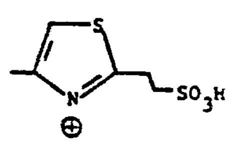 | $NH_2$ |
| 95 | " | 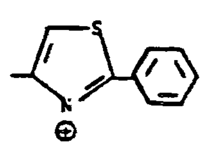 | " |

| | | | |
|---|---|---|---|
| 96 | " | 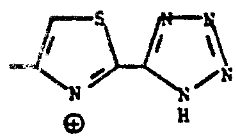 | " |
| 97 | $\overset{CH_3}{\mid}$ $-CHCH_2-$ | 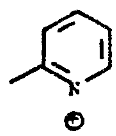 | $NH_2$ |
| 98 | $-CH_2-$ | 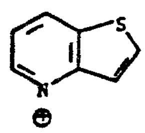 | " |
| 99 | $-CH_2-$ | 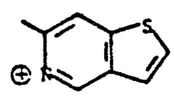 | $NH_2$ |
| 100 | " | 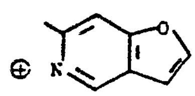 | " |
| 101 | " | 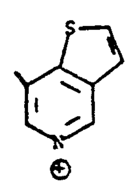 | " |

| | | | |
|---|---|---|---|
| 102 | " | 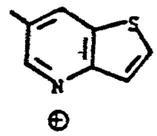 | " |
| 103 | " | 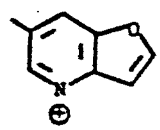 | " |
| 104 | " | 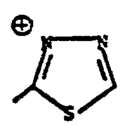 | " |
| 105 | $-CH_2-$ | 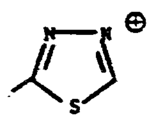 | $NH_2$ |
| 106 | " | 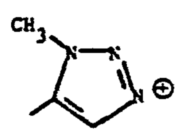 | " |
| 107 | $CH_2$ | 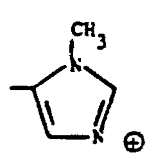 | $NH_2$ |

| | | | |
|---|---|---|---|
| 108 | " | 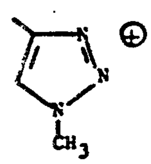 | " |
| 109 | " | 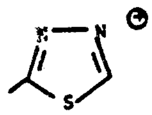 | " |
| 110 | " | 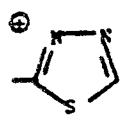 | " |
| 111 | " | 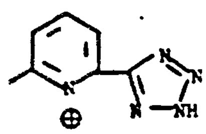 | " |
| 112 | $-CH_2-$ | 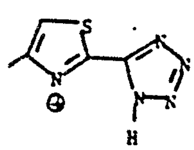 | $NH_2$ |
| 113 | " | 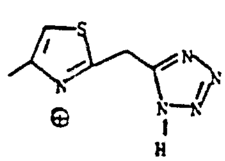 | " |

5. A process of Claim 1 wherein the compound is selected from the group consisting of

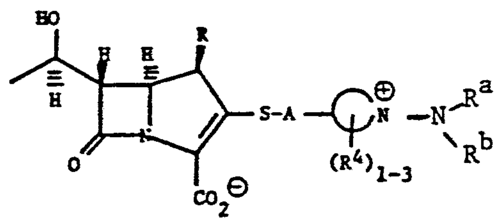

| Compound No. | A | 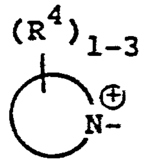 | 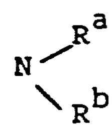 | $R_4$ |
|---|---|---|---|---|
| 1 | $CH_2$ | 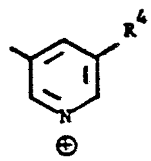 | $NH_2$ | $CO_2H$ |
| 2 | " | " | " | $CONH_2$ |
| 3 | " | " | " | CN |
| 4 | " | " | " | OH |
| 5 | " | " | " | $SO_2NH_2$ |
| 6 | " | " | " | $SO_3H$ |
| 7 | " | " | " | $NMe_2$ |
| 8 | " | " | " | $CONMe_2$ |

| | | | | |
|---|---|---|---|---|
| 9 | " | " | " | $CH_2NMe_2$ |
| 10 | " | " | " | $CH_2CN$ |
| 11 | " | " | " | $CH_2CONH_2$ |
| 12 | " | " | " | $CH_2CO_2H$ |
| 13 | " | " | " | $CH_2SCH_3$ |
| 14 | " | " | " | $CH_2SOCH_3$ |
| 15 | " | " | " | $CH_2SO_2CH_3$ |
| 16 | " | " | " | $SO_2CH_3$ |
| 17 | " | " | " | $SOCH_3$ |
| 18 | " | " | " | 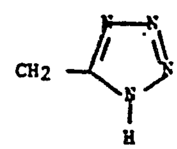 |
| 19 | " | " | " | $CH_2CH_2CO_2H$ |
| 20 | " | " | " | $CH_2SO_3H$ |
| 21 | " | " | " | $CH_2OCH_3$ |
| 22 | " | " | " | $CH_2\overset{\overset{O}{\uparrow}}{P}(-OH)OCH_3$ |

| | | | | |
|---|---|---|---|---|
| 23 | " | " | " | $CH_2CH_2SO_3H$ |
| 24 | " | " | " | $CF_3$ |
| 25 | " | " | " | $CH_2OC(=O)NH_2$ |
| 26 | " | " | " | $CH_2SO_2NH_2$ |
| 27 | " | " | " | Br |
| 28 | " | " | " | Cl |
| 29 | " | " | " | F |
| 30 | " | 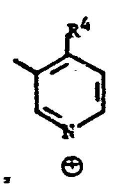 | " | $CO_2H$ |
| 31 | " | " | " | $CONH_2$ |
| 32 | " | " | " | CN |
| 33 | " | " | " | OH |
| 34 | " | " | " | $SONH_2$ |
| 35 | " | " | " | $SO_3H$ |
| 36 | " | " | " | $NMe_2$ |

| | | | | |
|---|---|---|---|---|
| 37 | " | " | " | $CONMe_2$ |
| 38 | " | " | " | $CH_2NMe_2$ |
| 39 | " | " | " | $CH_2CN$ |
| 40 | " | " | " | $CH_2CONH_2$ |
| 41 | " | " | " | $CH_2CO_2H$ |
| 41 | " | " | " | $CH_2SCH_3$ |
| 43 | " | " | " | $CH_2SOCH_3$ |
| 44 | " | " | " | $CH_2SO_2CH_3$ |
| 45 | " | " | " | $SO_2CH_3$ |
| 46 | " | " | " | $SOCH_3$ |
| 47 | " | " | " | 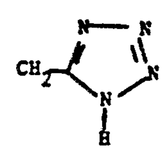 |
| 48 | " | " | " | $CH_2CH_2CO_2H$ |
| 49 | " | " | " | $CH_2SO_3H$ |
| 50 | " | " | " | $CH_2OCH_3$ |
| 51 | " | " | " | 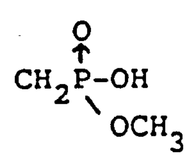 |

| | | | | |
|---|---|---|---|---|
| 52 | " | " | " | $CH_2CH_2SO_3H$ |
| 53 | " | " | " | $CF_3$ |
| 54 | " | " | " | $CH_2O\overset{O}{\overset{\|}{C}}NH_2$ |
| 55 | " | " | " | $CH_2SO_2NH_2$ |
| 56 | " | " | " | $CH_2SO_2NMe_2$ |
| 57 | " | 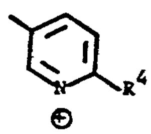 | " | $CO_2H$ |
| 58 | " | " | " | $CONH_2$ |
| 59 | " | " | " | CN |
| 60 | " | " | " | $OCH_3$ |
| 61 | " | " | " | $SO_2NH_2$ |
| 62 | " | " | " | $SO_3H$ |
| 63 | " | " | " | $NMe_2$ |
| 64 | " | " | " | $CONMe_2$ |
| 65 | " | " | " | $CH_2NMe_2$ |

| | | | | |
|---|---|---|---|---|
| 66 | " | " | " | $CH_2CN$ |
| 67 | " | " | " | $CH_2CONH_2$ |
| 68 | " | " | " | $CH_2CO_2H$ |
| 69 | " | " | " | $CH_2SCH_3$ |
| 70 | " | " | " | $CH_2SOCH_3$ |
| 71 | " | " | " | $CH_2SO_2CH_3$ |
| 72 | " | " | " | $SO_2CH_3$ |
| 73 | " | " | " | $SOCH_3$ |
| 74 | " | " | " | 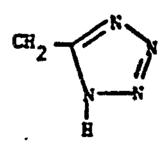 |
| 75 | " | " | " | $CH_2CH_2CO_2H$ |
| 76 | " | " | " | $CH_2SO_3H$ |
| 77 | " | " | " | $CH_2OCH_3$ |
| 78 | " | " | " | 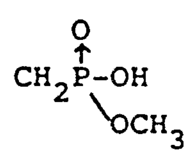 |
| 79 | " | " | " | $CH_2CH_2SO_3H$ |

| | | | | |
|---|---|---|---|---|
| 80 | " | " | " | $CF_3$ |
| 81 | " | " | " | $CH_2O\overset{O}{\overset{\|}{C}}NH_2$ |
| 82 | " | " | " | $CH_2SO_2NH_2$ |
| 83 | " | " | " | $CH_2SO_2NMe_2$ |
| 84 | " | " | " | 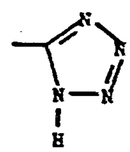 |
| 85 | " | 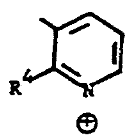 | " | $CO_2H$ |
| 86 | " | " | " | $CONH_2$ |
| 87 | " | " | " | CN |
| 88 | " | " | " | $OCH_3$ |
| 89 | " | " | " | $SO_2NH_2$ |
| 90 | " | " | " | $SO_3H$ |
| 91 | " | " | " | $NMe_2$ |
| 92 | " | " | " | $CONMe_2$ |

| | | | | |
|---|---|---|---|---|
| 93 | " | " | " | $CH_2NMe_2$ |
| 94 | " | " | " | $CH_2CN$ |
| 95 | " | " | " | $CH_2CONH_2$ |
| 96 | " | " | " | $CH_2CO_2H$ |
| 97 | " | " | " | $CH_2SCH_3$ |
| 98 | " | " | " | $CH_2SOCH_3$ |
| 99 | $CH_3-CH$ | " | " | $CH_2SO_2CH_3$ |
| 100 | " | " | " | $SO_2CH_3$ |
| 101 | " | " | " | $SOCH_3$ |
| 102 | " | " | " | 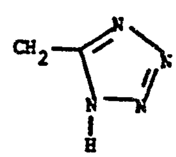 |
| 103 | " | " | " | $CH_2CH_2CO_2H$ |
| 104 | " | " | " | $CH_2SO_3H$ |
| 105 | " | " | " | $CH_2OCH_3$ |
| 106 | " | " | " | 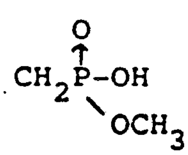 |

| | | | | |
|---|---|---|---|---|
| 107 | " | " | " | $CH_2CH_2SO_3H$ |
| 108 | " | " | " | $CF_3$ |
| 109 | " | " | " | $CH_2O\overset{O}{\overset{\|}{C}}NH_2$ |
| 110 | " | " | " | $CH_2SO_2NH_2$ |
| 111 | " | " | " | $CH_2SO_2NMe_2$ |
| 112 | " | " | " | 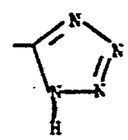 |
| 113 | $-CH_2-$ | 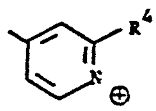 | " | $CO_2H$ |
| 114 | " | " | " | $CONH_2$ |
| 115 | " | " | " | CN |
| 116 | " | " | " | $OCH_3$ |
| 117 | " | " | " | $SO_2NH_2$ |
| 118 | " | " | " | $SO_3H$ |
| 119 | " | " | " | $NMe_2$ |

| | | | | |
|---|---|---|---|---|
| 120 | " | " | " | $CONMe_2$ |
| 121 | " | " | " | $CH_2NMe_2$ |
| 122 | " | " | " | $CH_2CN$ |
| 123 | " | " | " | $CH_2CONH_2$ |
| 124 | " | " | " | $CH_2CO_2H$ |
| 125 | " | " | " | $CH_2SCH_3$ |
| 126 | " | " | " | $CH_2SOCH_3$ |
| 127 | " | " | " | $CH_2SO_2CH_3$ |
| 128 | " | " | " | $SO_2CH_3$ |
| 129 | " | " | " | $SOCH_3$ |
| 130 | " | " | " | 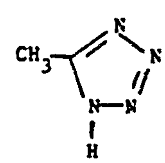 |
| 131 | " | " | " | $CH_2CH_2CO_2H$ |
| 132 | " | " | " | $CH_2SO_3H$ |
| 133 | " | " | " | $CH_2OCH_3$ |
| 134 | " | " | " | 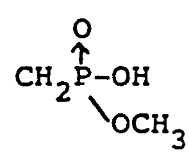 |

| | | | | |
|---|---|---|---|---|
| 135 | " | " | " | $CH_2CH_2SO_3H$ |
| 136 | " | " | " | $CF_3$ |
| 137 | " | " | " | $CH_2O\overset{O}{\overset{\|}{C}}NH_2$ |
| 138 | " | " | " | $CH_2SO_2NH_2$ |
| 139 | " | " | " | $CH_2SO_2NMe_2$ |
| 140 | " | " | " | 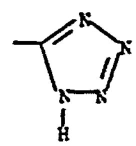 |
| 141 | " | 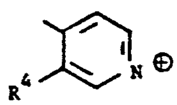 | " | $CO_2H$ |
| 142 | " | " | " | $CONH_2$ |
| 143 | " | " | " | CN |
| 144 | " | " | " | OH |
| 145 | " | " | " | $OCH_3$ |
| 146 | " | " | " | $SO_2NH_2$ |
| 147 | " | " | " | $SO_3H$ |

| | | | | |
|---|---|---|---|---|
| 148 | " | " | " | $NMe_2$ |
| 149 | " | " | " | $CONMe_2$ |
| 150 | " | " | " | $CH_2NMe_2$ |
| 151 | " | " | " | $CH_2CN$ |
| 152 | " | " | " | $CH_2CONH_2$ |
| 153 | " | " | " | $CH_2CO_2H$ |
| 154 | " | " | " | $CH_2SCH_3$ |
| 155 | " | " | " | $CH_2SOCH_3$ |
| 156 | " | " | " | $CH_2SO_2CH_3$ |
| 157 | " | " | " | $SO_2CH_3$ |
| 158 | " | " | " | $SOCH_3$ |
| 159 | " | " | " | 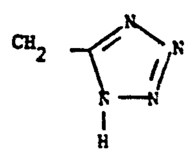 |
| 160 | " | " | " | $CH_2CH_2CO_2H$ |
| 161 | " | " | " | $CH_2SO_3H$ |
| 162 | " | " | " | $CH_2OCH_3$ |

| | | | | |
|---|---|---|---|---|
| 163 | " | " | " | 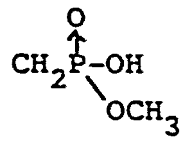 |
| 164 | " | " | " | $CH_2CH_2SO_3H$ |
| 165 | " | " | " | $CF_3$ |
| 166 | " | " | " | $CH_2O\overset{O}{\overset{\|}{C}}NH_2$ |
| 167 | " | " | " | $CH_2SO_2NH_2$ |
| 168 | " | " | " | $CH_2SO_2NMe_2$ |
| 169 | " | " | " | 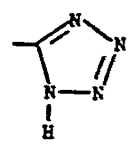 |
| 170 | " | " | " | F |
| 171 | " | " | " | Cl |
| 172 | " | " | " | Br |
| 173 | " | 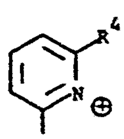 | " | $CO_2H$ |
| 174 | " | " | " | $CONH_2$ |

| | | | | |
|---|---|---|---|---|
| 175 | " | " | " | CN |
| 176 | " | " | " | $SO_2NH_2$ |
| 177 | " | " | " | $SO_3H$ |
| 178 | " | " | " | $NMe_2$ |
| 179 | " | " | " | $CONMe_2$ |
| 180 | " | " | " | $CH_2NMe_2$ |
| 181 | " | " | " | $CH_2CN$ |
| 182 | " | " | " | $CH_2CONH_2$ |
| 183 | " | " | " | $CH_2CO_2H$ |
| 184 | " | " | " | $CH_2SCH_3$ |
| 185 | " | " | " | $CH_2SOCH_3$ |
| 186 | " | " | " | $CH_2SO_2CH_3$ |
| 187 | " | " | " | $SO_2CH_3$ |
| 188 | " | " | " | 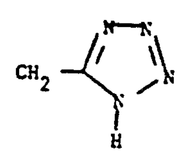 |
| 189 | " | " | " | $CH_2CH_2CO_2H$ |

| | | | | |
|---|---|---|---|---|
| 190 | " | " | " | $CH_2SO_3H$ |
| 191 | " | " | " | $CH_2OCH_3$ |
| 192 | " | " | " | 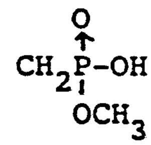 |
| 193 | " | " | " | $CH_2CH_2SO_3H$ |
| 194 | " | " | " | $CF_3$ |
| 195 | " | " | " | 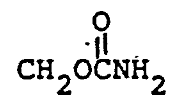 |
| 196 | " | " | " | $CH_2SO_2NH_2$ |
| 197 | " | 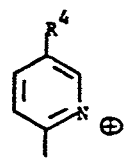 | " | $CO_2H$ |
| 198 | " | " | " | $CONH_2$ |
| 199 | " | " | " | CN |
| 200 | " | " | " | OH |
| 201 | " | " | " | $SO_2NH_2$ |
| 202 | " | " | " | $SO_3H$ |

| | | | | |
|---|---|---|---|---|
| 203 | " | " | " | $NMe_2$ |
| 204 | " | " | " | $CONMe_2$ |
| 205 | " | " | " | $CH_2NMe_2$ |
| 206 | " | " | " | $CH_2CN$ |
| 207 | " | " | " | $CH_2CONH_2$ |
| 208 | " | " | " | $CH_2CO_2H$ |
| 209 | " | " | " | $CH_2SCH_3$ |
| 210 | " | " | " | $CH_2SOCH_3$ |
| 211 | " | " | " | $CH_2SO_2CH_3$ |
| 212 | " | " | " | $SO_2CH_3$ |
| 213 | " | " | " | $SOCH_3$ |
| 214 | " | " | " | 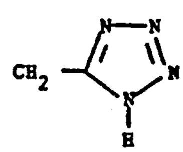 |
| 215 | " | " | " | $CH_2CH_2CO_2H$ |
| 216 | " | " | " | $CH_2SO_3H$ |
| 217 | " | " | " | $CH_2OCH_3$ |

| | | | | |
|---|---|---|---|---|
| 218 | " | " | " | 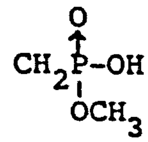 |
| 219 | " | " | " | $CH_2CH_2SO_3H$ |
| 220 | " | " | " | $CF_3$ |
| 221 | " | " | " | 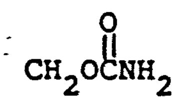 |
| 222 | " | " | " | $CH_2SO_2NH_2$ |
| 223 | " | " | " | Br |
| 224 | " | " | " | Cl |
| 225 | " | " | " | F |
| 226 | " | 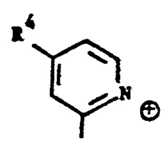 | " | $CO_2H$ |
| 227 | " | " | " | $CONH_2$ |
| 228 | " | " | " | CN |
| 229 | " | " | " | $SO_2NH_2$ |
| 230 | " | " | " | $SO_3H$ |

| | | | | |
|---|---|---|---|---|
| 231 | " | " | " | $NMe_2$ |
| 232 | " | " | " | $CONMe_2$ |
| 233 | " | " | " | $CH_2NMe_2$ |
| 234 | " | " | " | $CH_2CN$ |
| 235 | " | " | " | $CH_2CONH_2$ |
| 236 | " | " | " | $CH_2CO_2H$ |
| 237 | " | " | " | $CH_2SCH_3$ |
| 238 | " | " | " | $CH_2SOCH_3$ |
| 239 | " | " | " | $CH_2SO_2CH_3$ |
| 240 | " | " | " | $SO_2CH_3$ |
| 241 | " | " | " | $SOCH_3$ |
| 242 | " | " | " | 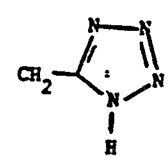 |
| 243 | " | " | " | $CH_2CH_2CO_2H$ |
| 244 | " | " | " | $CH_2SO_3H$ |
| 245 | " | " | " | $CH_2OCH_3$ |

| | | | | |
|---|---|---|---|---|
| 246 | " | " | " | 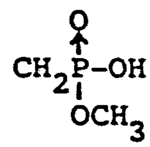 |
| 247 | " | " | " | $CH_2CH_2SO_3H$ |
| 248 | " | " | " | $CF_3$ |
| 249 | " | " | " | 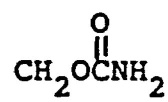 |
| 250 | " | " | " | $CH_2SO_2NH_2$ |
| 251 | " | 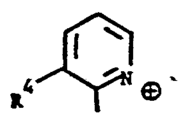 | " | $CO_2H$ |
| 252 | " | " | " | $CONH_2$ |
| 253 | " | " | " | CN |
| 254 | " | " | " | OH |
| 255 | " | " | " | $SO_2NH_2$ |
| 256 | " | " | " | $SO_3H$ |
| 257 | " | " | " | $NMe_2$ |
| 258 | " | " | " | $CONMe_2$ |

| | | | | |
|---|---|---|---|---|
| 259 | " | " | " | $CH_2NMe_2$ |
| 260 | " | " | " | $CH_2CN$ |
| 261 | " | " | " | $CH_2CONH_2$ |
| 262 | " | " | " | $CH_2CO_2H$ |
| 263 | " | " | " | $CH_2SCH_3$ |
| 264 | " | " | " | $CH_2SOCH_3$ |
| 265 | " | " | " | $CH_2SO_2CH_3$ |
| 266 | " | " | " | $SO_2CH_3$ |
| 267 | " | " | " | $SOCH_3$ |
| 268 | " | " | " | 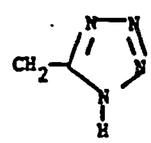 |
| 269 | " | " | " | $CH_2CH_2CO_2H$ |
| 270 | " | " | " | $CH_2SO_3H$ |
| 271 | " | " | " | $CH_2OCH_3$ |
| 272 | " | " | " | 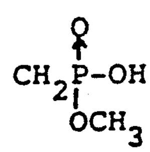 |

| | | | | |
|---|---|---|---|---|
| 273 | " | " | " | $CH_2CH_2SO_3H$ |
| 274 | " | " | " | $CF_3$ |
| 275 | " | " | " | $CH_2O\overset{O}{\overset{\|}{C}}NH_2$ |
| 276 | " | " | " | $CH_2SO_2NH_2$ |
| 277 | " | " | " | Br |
| 278 | " | " | " | Cl |
| 279 | " | " | " | F |
| 280 | $CH_2$ | 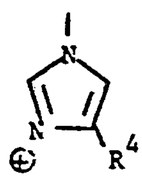 | $NH_2$ | COOH |
| 281 | " | " | " | $CONH_2$ |
| 282 | " | " | " | $CH_2OH$ |
| 283 | " | " | " | $CH_2SO_3H$ |
| 284 | $CH_2$ | 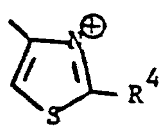 | " | COOH |
| 285 | " | " | " | $CONH_2$ |
| 286 | " | " | " | $CH_2COOH$ |

| | | | | |
|---|---|---|---|---|
| 287 | " | " | " | $CH_2SO_3H$ |
| 288 | " | " | " | $CH_2CN$ |
| 289 | $CH_2$ | 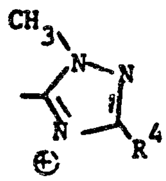 | $NH_2$ | $CH_2SO_3H$ |
| 290 | " | " | " | $CH_2COOH$- |
| 291 | " | " | " | COOH |
| 292 | " | " | " | $CONH_2$ |
| 293 | " | " | " | $CH_2OH$ |

6. A process for producing a pharmaceutical composition for antibacterial use comprising mixing an antibacterially effective amount of a compound prepared as claimed in Claim 1 and a pharmaceutically acceptable carrier.

7. A process which comprises combining a compound of Claim 1 and a dehydropeptidase inhibitor.

8. A process for preparing a pharmaceutical composition for antibacterial use comprising combining an antibacterially effective amount of a compound of Claim 1, an inhibitorily effective amount of a dehydropeptidase inhibitor, and, optionally, a pharmaceutically acceptable carrier.